# EUROPÄISCHE PATENTSCHRIFT

(11) **EP 1 191 100 B1**
(45) Veröffentlichungstag und Bekanntmachung des Hinweises auf die Patenterteilung: **17.03.2010**
(21) Anmeldenummer: 01120286.8
(22) Anmeldetag: 23.08.2001
(51) Int. Cl.: C12N 15/12, C07K 14/435, C12N 9/02, C12N 9/12, C12N 15/63, C12N 15/62, C07K 16/18, C07K 16/40, C12Q 1/68, G01N 33/53, A61K 39/35, A61K 39/395

(54) **Rekombinante Allergene aus der Dörrobstmotte Plodia interpunctella**
Recombinant allergens from the Indianmeal Moth Plodia interpunctella
Allergènes de recombinaison derivés de la mite Plodia interpunctella

(30) Priorität: 24.08.2000 DE 10041541
(43) Veröffentlichungstag der Anmeldung: 27.03.2002
(73) Patentinhaber: Duchene, Michael, Dr., 1070 Wien (AT)
(72) Erfinder: Duchene, Michael, 1070 Wien (AT); Binder, Marina, 1140 Wien (AT); Mahler, Vera, 91054 Erlangen (DE); Hayek, Brigitte, 1180 Wien (AT); Prozell, Sabine, 10407 Berlin (DE); Schöller, Matthias, 10247 Berlin (DE)
(74) Vertreter: Weiss, Wolfgang

(56) Entgegenhaltungen:
- LIN, R.Y. ET AL.: "Identification and characterization of a 30 kd major allergen from Parapenaeus fissurus" JOURNAL OF ALLERGY AND CLINICAL IMMUNOLOGY, Bd. 92, Nr. 6, Dezember 1993 (1993-12), Seiten 837-845, XP000926734
- HENWOOD, B.P. & MACDONALD, D.M.: "Caterpillar dermatitis" CLINICAL AND EXPERIMENTAL DERMATOLOGY, Bd. 8, Nr. 1, Januar 1983 (1983-01), Seiten 77-93, XP000926742
- DUMAS, C. & CAMONIS, J.: "Cloning and Sequence Analysis of the cDNA for Arginine Kibase of Lobster Muscle" JOURNAL OF BIOLOGICAL CHEMISTRY, Bd. 268, Nr. 29, 15. Oktober 1993 (1993-10-15), Seiten 21599-21605, XP002188399
- BALDO, B.A. & PANZANI, R.C.: "Detection of IgE Antibodies to a Wide Range of Insect Species in Subjects with Suspected Inhalant Allergies to Insects" INTERNATIONAL ARCHIVES OF ALLERGY AND APPLIED IMMUNOLOGY, Bd. 85, Nr. 3, 1988, Seiten 278-287, XP000926736
- REESE, G. ET AL.: "Tropomyosin: An Invertebrate Pan-Allergen" INTERNATIONAL ARCHIVES OF ALLERGY AND IMMUNOLOGY, Bd. 119, Nr. 4, August 1999 (1999-08), Seiten 247-258, XP000926735
- WANG, Y.E. ET AL.: "Arginine Kinase Expression and Localization in Growth Cone Migration" JOURNAL OF NEUROSCIENCE, Bd. 18, Nr. 3, 1. Februar 1998 (1998-02-01), Seiten 987-998, XP002188400
- BINDER, M. ET AL.: "Molecular and Immunological Characterization of Arginine Kinase from the Indianmeal Moth, Plodia interpunctella, a Novel Cross-Reactive Invertebrate Pan-Allergen" JOURNAL OF IMMUNOLOGY, Bd. 167, Nr. 9, 1. November 2001 (2001-11-01), Seiten 5470-5477, XP002188401
- THE FOOD INSECTS NEWSLETTER, [Online] Bd. VIII, Nr. 2, Juli 1995 (1995-07), Gefunden im Internet: URL:http://www.food-insects.com/Vol8%20no2 .htm> [gefunden am 2007-06-11]
- DATABASE EMBL 16. März 2000 (2000-03-16), WEICHRAUCH D., TOWLE D.W.: "Eriocheir sinensis arginine kinase mRNA" Database accession no. AF233356

### SEQUENZPROTOKOLL

<110> Duchene, Michael
<120> Rekombinante Allergene aus der Motte Plodia interpunctella
<130> 22034pdemd
<140>
   <141>
<160> 8
<170> PatentIn Ver. 2.1
<210> 1
   <211> 1294
   <212> DNA
   <213> Plodia interpunctella
<220>
   <221> CDS
   <222> (25)..(1089)
<400> 1
   tcaagtgtca gaaaagcagc agca atg gtg gac gcc gct acc ctt gag aaa     51
                              Met Val Asp Ala Ala Thr Leu Glu Lys
                               1               5
   ttg gag gct ggc ttc agc aag ctt gcc gcc tcc gac tca aag tcg ctg     99
   Leu Glu Ala Gly Phe Ser Lys Leu Ala Ala Ser Asp Ser Lys Ser Leu
    10                  15                  20                  25
   ctg aag aaa tac ctc acc agg gag gta ttt gat gct ctc aag aac aag    147
   Leu Lys Lys Tyr Leu Thr Arg Glu Val Phe Asp Ala Leu Lys Asn Lys
                    30                  35                  40
   aag acc tca ttt ggt tca act ctc ctg gat tct atc cag tca ggt gtt    195
   Lys Thr Ser Phe Gly Ser Thr Leu Leu Asp Ser Ile Gln Ser Gly Val
                45                  50                  55
   gag aac tta cat tcg ggt gtt gga att tat gcc cca gat gct gag gca    243
   Glu Asn Leu His Ser Gly Val Gly Ile Tyr Ala Pro Asp Ala Glu Ala
            60                  65                  70
   tat gta gta ttt gca gac ttg ttc gac ccc atc att gaa gat tac cac    291
   Tyr Val Val Phe Ala Asp Leu Phe Asp Pro Ile Ile Glu Asp Tyr His
   75 80 85
   aat ggc ttc aag aaa acc gac aag cac cct ccc aag aac tgg gga gat 339
   Asn Gly Phe Lys Lys Thr Asp Lys His Pro Pro Lys Asn Trp Gly Asp
   90 95 100 105
   gtt gag acc ctc ggg aac ttg gat cct gct ggt gaa ttt gtt gtc tcc 387
   Val Glu Thr Leu Gly Asn Leu Asp Pro Ala Gly Glu Phe Val Val Ser
   110 115 120
   acc cgt gtc cgc tgc ggt cgc tcc atg gaa ggc tac cca ttc aac ccc 435
   Thr Arg Val Arg Cys Gly Arg Ser Met Glu Gly Tyr Pro Phe Asn Pro
   125 130 135
   tgc tta aca gag gcc caa tac aag gaa atg gaa gag aaa gtc tcc tcc 483
   Cys Leu Thr Glu Ala Gln Tyr Lys Glu Met Glu Glu Lys Val Ser Ser
   140 145 150
   aca ctc tcc ggc ctc gag ggt gaa ctg aaa ggc acc ttt ttc cca ctc 531
   Thr Leu Ser Gly Leu Glu Gly Glu Leu Lys Gly Thr Phe Phe Pro Leu
   155 160 165
   aca ggc atg tcc aag gag act caa caa cag ttg att gat gac cac ttc 579
   Thr Gly Met Ser Lys Glu Thr Gln Gln Gln Leu Ile Asp Asp His Phe
   170 175 180 185
   ctg ttc aag gag ggt gat cgc ttc ctc cag gcc gct aac gct tgc cgc 627
   Leu Phe Lys Glu Gly Asp Arg Phe Leu Gln Ala Ala Asn Ala Cys Arg
   190 195 200
   ttc tgg ccc tcc ggt cgt ggc atc tac cac aat gag aac aag act ttc 675
   Phe Trp Pro Ser Gly Arg Gly Ile Tyr His Asn Glu Asn Lys Thr Phe
   205 210 215
   ctg gta tgg tgc aat gag gag gac cac ctc cgt ctg atc tcc atg caa 723
   Leu Val Trp Cys Asn Glu Glu Asp His Leu Arg Leu Ile Ser Met Gln
   220 225 230
   atg ggc ggc gac ctg aag cag gtg tac aag agg ctg gtg agg gga gtg 771
   Met Gly Gly Asp Leu Lys Gln Val Tyr Lys Arg Leu Val Arg Gly Val
   235 240 245
   aac gac atc gcg aag agg atc cca ttc tcg cac aac gag cgg ctg ggc 819
   Asn Asp Ile Ala Lys Arg Ile Pro Phe Ser His Asn Glu Arg Leu Gly
   250 255 260 265
   ttc ctg act ttc tgc ccc acc aac ctg ggc aca acg gtg cgc gca tcg 867
   Phe Leu Thr Phe Cys Pro Thr Asn Leu Gly Thr Thr Val Arg Ala Ser
   270 275 280
   gtg cac atc aag ctg ccc aag ctg gcg gcc gac aag gcc aag ctg gag 915
   Val His Ile Lys Leu Pro Lys Leu Ala Ala Asp Lys Ala Lys Leu Glu
   285 290 295
   gag gtg gcc agc aag tac cac ctg cag gtg cgc ggc acc cgc ggc gag 963
   Glu Val Ala Ser Lys Tyr His Leu Gln Val Arg Gly Thr Arg Gly Glu
   300 305 310
   cac acg gag gcc gag ggc ggc gtc tac gac atc tcc aac aag agg cgc 1011
   His Thr Glu Ala Glu Gly Gly Val Tyr Asp Ile Ser Asn Lys Arg Arg
   315 320 325
   atg gga ctc acc gag tac gaa gcc gtc aag gag atg tac gac ggc atc 1059
   Met Gly Leu Thr Glu Tyr Glu Ala Val Lys Glu Met Tyr Asp Gly Ile
   330 335 340 345
   gct gaa ctg atc aaa atc gag aaa tcc ctg taagatgttt aacgatctcg 1109
   Ala Glu Leu Ile Lys Ile Glu Lys Ser Leu
   350 355
   cgctatcagt attttttgta ttatttatcg ttttcacata agtattggat gtgaaggggc 1169
   gagggcgaca ctagtcagcg gccttgagcg gggccgggca cgcgggcggc ccactatact 1229
   gtttcgtaaa agtattgtct ataaggaaat ggaaaataaa gacagctagc gttaagacaa 1289
   aaaaa 1294
<210> 2
   <211> 355
   <212> PRT
   <213> Plodia interpunctella
<400> 2
   Met Val Asp Ala Ala Thr Leu Glu Lys Leu Glu Ala Gly Phe Ser Lys
   1 5 10 15
   Leu Ala Ala Ser Asp Ser Lys Ser Leu Leu Lys Lys Tyr Leu Thr Arg
   20 25 30
   Glu Val Phe Asp Ala Leu Lys Asn Lys Lys Thr Ser Phe Gly Ser Thr
   35 40 45
   Leu Leu Asp Ser Ile Gln Ser Gly Val Glu Asn Leu His Ser Gly Val
   50 55 60
   Gly Ile Tyr Ala Pro Asp Ala Glu Ala Tyr Val Val Phe Ala Asp Leu
   65 70 75 80
   Phe Asp Pro Ile Ile Glu Asp Tyr His Asn Gly Phe Lys Lys Thr Asp
   85 90 95
   Lys His Pro Pro Lys Asn Trp Gly Asp Val Glu Thr Leu Gly Asn Leu
   100 105 110
   Asp Pro Ala Gly Glu Phe Val Val Ser Thr Arg Val Arg Cys Gly Arg
   115 120 125
   Ser Met Glu Gly Tyr Pro Phe Asn Pro Cys Leu Thr Glu Ala Gln Tyr
   130 135 140
   Lys Glu Met Glu Glu Lys Val Ser Ser Thr Leu Ser Gly Leu Glu Gly
   145 150 155 160
   Glu Leu Lys Gly Thr Phe Phe Pro Leu Thr Gly Met Ser Lys Glu Thr
   165 170 175
   Gln Gln Gln Leu Ile Asp Asp His Phe Leu Phe Lys Glu Gly Asp Arg
   180 185 190
   Phe Leu Gln Ala Ala Asn Ala Cys Arg Phe Trp Pro Ser Gly Arg Gly
   195 200 205
   Ile Tyr His Asn Glu Asn Lys Thr Phe Leu Val Trp Cys Asn Glu Glu
   210 215 220
   Asp His Leu Arg Leu Ile Ser Met Gln Met Gly Gly Asp Leu Lys Gln
   225 230 235 240
   Val Tyr Lys Arg Leu Val Arg Gly Val Asn Asp Ile Ala Lys Arg Ile
   245 250 255
   Pro Phe Ser His Asn Glu Arg Leu Gly Phe Leu Thr Phe Cys Pro Thr
   260 265 270
   Asn Leu Gly Thr Thr Val Arg Ala Ser Val His Ile Lys Leu Pro Lys
   275 280 285
   Leu Ala Ala Asp Lys Ala Lys Leu Glu Glu Val Ala Ser Lys Tyr His
   290 295 300
   Leu Gln Val Arg Gly Thr Arg Gly Glu His Thr Glu Ala Glu Gly Gly
   305 310 315 320
   Val Tyr Asp Ile Ser Asn Lys Arg Arg Met Gly Leu Thr Glu Tyr Glu
                   325                 330                 335
   Ala Val Lys Glu Met Tyr Asp Gly Ile Ala Glu Leu Ile Lys Ile Glu
               340                 345                 350
   Lys Ser Leu
           355
<210> 3
   <211> 1092
   <212> DNA
   <213> Plodia interpunctella
<220>
   <221> CDS
   <222> (31)..(885)
<400> 3
   acaggacagt agacacacaa agccaccacc atg gac gcg atc aag aag aag atg    54
                                    Met Asp Ala Ile Lys Lys Lys Met
                                      1               5
   cag gcg atg aag ctg gag aag gac aac gct ttg gac cgc gct gcc atg    102
   Gln Ala Met Lys Leu Glu Lys Asp Asn Ala Leu Asp Arg Ala Ala Met
        10                  15                  20
   tgc gag cag cag gcc aag gac gcc aac ctc cgt gct gag aag gcc gag    150
   Cys Glu Gln Gln Ala Lys Asp Ala Asn Leu Arg Ala Glu Lys Ala Glu
    25                  30                  35                  40
   gag gag gcc aga caa ttg cag aag aag atc cag acg att gag aac gat    198
   Glu Glu Ala Arg Gln Leu Gln Lys Lys Ile Gln Thr Ile Glu Asn Asp
                    45                  50                  55
   ctg gac cag acg cag gag gcg ctc atg cag gtc aac gcc aag ctg gaa    246
   Leu Asp Gln Thr Gln Glu Ala Leu Met Gln Val Asn Ala Lys Leu Glu
                60                  65                  70
   gag aaa gag aaa gct ctt cag aac gct gag tcc gaa gtc gct gcc ctc    294
   Glu Lys Glu Lys Ala Leu Gln Asn Ala Glu Ser Glu Val Ala Ala Leu
            75                  80                  85
   aac cga cgt atc caa ctg ctg gaa gag gac ctc gag agg tcc gag gag    342
   Asn Arg Arg Ile Gln Leu Leu Glu Glu Asp Leu Glu Arg Ser Glu Glu
               90                  95                  100
   cgc ctc gcc acc gcc aca gcc aaa ctg tcc gaa gcc agc cag gct gcc   390
   Arg Leu Ala Thr Ala Thr Ala Lys Leu Ser Glu Ala Ser Gln Ala Ala
   105                 110                 115                 120
   gat gag tcg gaa cgt gcc cgc aag gtg ctc gag aac agg tca ttg gct   438
   Asp Glu Ser Glu Arg Ala Arg Lys Val Leu Glu Asn Arg Ser Leu Ala
                   125                 130                 135
   gat gaa gag cgt atg gac gct ttg gag aac cag ctg aag gaa gcc agg   486
   Asp Glu Glu Arg Met Asp Ala Leu Glu Asn Gln Leu Lys Glu Ala Arg
               140                 145                 150
   ttc ctt gct gag gaa gcc gac aag aaa tac gat gag gtt gct cgt aag   534
   Phe Leu Ala Glu Glu Ala Asp Lys Lys Tyr Asp Glu Val Ala Arg Lys
           155                 160                 165
   ctg gcc atg gtt gag gct gac ctg gag cgc gcg gag gag cgt gcc gaa   582
   Leu Ala Met Val Glu Ala Asp Leu Glu Arg Ala Glu Glu Arg Ala Glu
       170                 175                 180
   tcc ggc gaa tcc aaa atc gtc gag ctt gag gaa gaa ctg cgc gtg gtt   630
   Ser Gly Glu Ser Lys Ile Val Glu Leu Glu Glu Glu Leu Arg Val Val
   185                 190                 195                 200
   ggc aac aac ttg aaa tcc ctg gaa gtc tcc gag gag aag gcc aac caa   678
   Gly Asn Asn Leu Lys Ser Leu Glu Val Ser Glu Glu Lys Ala Asn Gln
                   205                 210                 215
   cgt gag gag gag tac aaa aat cag atc aaa acc ctc acc acc cgc cta   726
   Arg Glu Glu Glu Tyr Lys Asn Gln Ile Lys Thr Leu Thr Thr Arg Leu
               220                 225                 230
   aag gag gct gag gcc cgc gct gag ttc gcc gag cgt tcc gtg cag aaa   774
   Lys Glu Ala Glu Ala Arg Ala Glu Phe Ala Glu Arg Ser Val Gln Lys
           235                 240                 245
   ctg caa aag gag gtc gac agg ctt gaa gac gaa ctg gtg gct gag aag   822
   Leu Gln Lys Glu Val Asp Arg Leu Glu Asp Glu Leu Val Ala Glu Lys
       250                 255                 260
   gag aaa tac aaa gat att ggt gac gac ctg gac acc ccc ttc gtc gag   870
   Glu Lys Tyr Lys Asp Ile Gly Asp Asp Leu Asp Thr Pro Phe Val Glu
   265                 270                 275                 280
   ctc atc ctc aag gaa taaactcctc acgttggtca cctgggcctg tcccatgcgg   925
   Leu Ile Leu Lys Glu
   285
   ggcagaccca cgggtcattc caagacgcgg ctcttccgcc agcgattcaa catctgtaca 985
   gatgttatat tcattttata ctcatttaaa atatttaaat ctatagtttt atggcggtat 1045
   ttattttcga gtaatataat aaataattta ttacttattt aaaaaaa 1092
<210> 4
   <211> 285
   <212> PRT
   <213> Plodia interpunctella
<400> 4
   Met Asp Ala Ile Lys Lys Lys Met Gln Ala Met Lys Leu Glu Lys Asp
    1               5                  10                  15
   Asn Ala Leu Asp Arg Ala Ala Met Cys Glu Gln Gln Ala Lys Asp Ala
               20                  25                  30
   Asn Leu Arg Ala Glu Lys Ala Glu Glu Glu Ala Arg Gln Leu Gln Lys
           35                  40                  45
   Lys Ile Gln Thr Ile Glu Asn Asp Leu Asp Gln Thr Gln Glu Ala Leu
       50                  55                  60
   Met Gln Val Asn Ala Lys Leu Glu Glu Lys Glu Lys Ala Leu Gln Asn
   65                  70                  75                  80
   Ala Glu Ser Glu Val Ala Ala Leu Asn Arg Arg Ile Gln Leu Leu Glu
                   85                  90                  95
   Glu Asp Leu Glu Arg Ser Glu Glu Arg Leu Ala Thr Ala Thr Ala Lys
               100                 105                 110
   Leu Ser Glu Ala Ser Gln Ala Ala Asp Glu Ser Glu Arg Ala Arg Lys
           115                 120                 125
   Val Leu Glu Asn Arg Ser Leu Ala Asp Glu Glu Arg Met Asp Ala Leu
       130                 135                 140
   Glu Asn Gln Leu Lys Glu Ala Arg Phe Leu Ala Glu Glu Ala Asp Lys
   145                 150                 155                 160
   Lys Tyr Asp Glu Val Ala Arg Lys Leu Ala Met Val Glu Ala Asp Leu
                   165                 170                 175
   Glu Arg Ala Glu Glu Arg Ala Glu Ser Gly Glu Ser Lys Ile Val Glu
               180                     185                     190
   Leu Glu Glu Glu Leu Arg Val Val Gly Asn Asn Leu Lys Ser Leu Glu
           195                     200                     205
   Val Ser Glu Glu Lys Ala Asn Gln Arg Glu Glu Glu Tyr Lys Asn Gln
       210                     215                     220
   Ile Lys Thr Leu Thr Thr Arg Leu Lys Glu Ala Glu Ala Arg Ala Glu
   225                     230                     235                     240
   Phe Ala Glu Arg Ser Val Gln Lys Leu Gln Lys Glu Val Asp Arg Leu
                   245                     250                     255
   Glu Asp Glu Leu Val Ala Glu Lys Glu Lys Tyr Lys Asp Ile Gly Asp
               260                     265                     270
   Asp Leu Asp Thr Pro Phe Val Glu Leu Ile Leu Lys Glu
           275                     280                     285
<210> 5
   <211> 2230
   <212> DNA
   <213> Plodia interpunctella
<220>
   <221> CDS
   <222> (13)..(2127)
<400> 5
   ggtgggtgga cg atg aag act gtc ctg atc tta gct ggc ctc gtg gcc ctg 51
                 Met Lys Thr Val Leu Ile Leu Ala Gly Leu Val Ala Leu
                   1               5                   10
   gcc gcg ggc aac acc ttc ccg gta ttc aga tat gac cac gtc gaa act    99
   Ala Ala Gly Asn Thr Phe Pro Val Phe Arg Tyr Asp His Val Glu Thr
        15                  20                  25
   aga aaa ttg gaa gga gac ctt tta cag tac cag tcg aaa ttt ctg tct   147
   Arg Lys Leu Glu Gly Asp Leu Leu Gln Tyr Gln Ser Lys Phe Leu Ser
    30                  35                  40                  45
   ctt ctt gag aat gtg aga cag att gac tac gaa gcg gag tac tac aaa   195
   Leu Leu Glu Asn Val Arg Gln Ile Asp Tyr Glu Ala Glu Tyr Tyr Lys
                    50                  55                  60
   gtt ggc aag ggt tac gac atc gta gcc agc ata gag aac tat tct gac    243
   Val Gly Lys Gly Tyr Asp Ile Val Ala Ser Ile Glu Asn Tyr Ser Asp
                   65                  70                  75
   caa gat gca gtc agg gcg ttt gct ggt ctt cga gaa att ggt ttc atg    291
   Gln Asp Ala Val Arg Ala Phe Ala Gly Leu Arg Glu Ile Gly Phe Met
               80                  85                  90
   ccc aaa gct tac aca ttc tcc att ttc tac gac agg cag aga gaa gaa    339
   Pro Lys Ala Tyr Thr Phe Ser Ile Phe Tyr Asp Arg Gln Arg Glu Glu
           95                  100                 105
   gct aag att att tat gac ttg ttc tac agc gct aaa gat ttg gac act    387
   Ala Lys Ile Ile Tyr Asp Leu Phe Tyr Ser Ala Lys Asp Leu Asp Thr
   110                 115                 120                 125
   ttc tac aag act gta gcc tac ggc cga atc tat ttc aac gag tat cag    435
   Phe Tyr Lys Thr Val Ala Tyr Gly Arg Ile Tyr Phe Asn Glu Tyr Gln
                   130                 135                 140
   ttc atg tat gct ttc tat gct gcg att att cag cgc tct gat acc aca    483
   Phe Met Tyr Ala Phe Tyr Ala Ala Ile Ile Gln Arg Ser Asp Thr Thr
                   145                 150                 155
   gga atc gtc tta cca gct cca tat gaa ctg tat cct gaa tat ttc ttg    531
   Gly Ile Val Leu Pro Ala Pro Tyr Glu Leu Tyr Pro Glu Tyr Phe Leu
               160                 165                 170
   aac atg tat acg atc caa aga atg tac cga aca cag atg caa agt ggt    579
   Asn Met Tyr Thr Ile Gln Arg Met Tyr Arg Thr Gln Met Gln Ser Gly
           175                 180                 185
   ata ttc aat gag gaa gtt gct agt aac tat ggt atc tgg aag atg gat    627
   Ile Phe Asn Glu Glu Val Ala Ser Asn Tyr Gly Ile Trp Lys Met Asp
   190                 195                 200                 205
   aat aac tac tat tat tac tac aac tac tct aat ccc ttg acg tac aga    675
   Asn Asn Tyr Tyr Tyr Tyr Tyr Asn Tyr Ser Asn Pro Leu Thr Tyr Arg
                   210                 215                 220
   aat cag gag tac aga ttg tct tat ttg aca gaa gac ata ggc tgg aac    723
   Asn Gln Glu Tyr Arg Leu Ser Tyr Leu Thr Glu Asp Ile Gly Trp Asn
                   225                 230                 235
   tct tac tat tac tac ttc cac aat ctt atg cct ttc tgg ggc aaa ggc    771
   Ser Tyr Tyr Tyr Tyr Phe His Asn Leu Met Pro Phe Trp Gly Lys Gly
               240                 245                 250
   gag gac ttt att ggt atc ttc aag gaa cgc cgt gga gaa ttc tac tac    819
   Glu Asp Phe Ile Gly Ile Phe Lys Glu Arg Arg Gly Glu Phe Tyr Tyr
       255                 260                 265
   tac ttc tat cag caa ctc ttg tct cgt tac tac ctt gag cgt ttg agt    867
   Tyr Phe Tyr Gln Gln Leu Leu Ser Arg Tyr Tyr Leu Glu Arg Leu Ser
   270                 275                 280                 285
   aat ggc ttg gga gaa att cca gat ttc tct tgg tac caa cct ctg agg    915
   Asn Gly Leu Gly Glu Ile Pro Asp Phe Ser Trp Tyr Gln Pro Leu Arg
                   290                 295                 300
   agt ggt tac tat cca gct ata tat acg agc tca gcc tat ccg ttt gct    963
   Ser Gly Tyr Tyr Pro Ala Ile Tyr Thr Ser Ser Ala Tyr Pro Phe Ala
               305                 310                 315
   caa cgt ccc aac tat tat tac atg gga act gaa gaa aat gtt gac tac    1011
   Gln Arg Pro Asn Tyr Tyr Tyr Met Gly Thr Glu Glu Asn Val Asp Tyr
           320                 325                 330
   atc caa ttc ctt gat gct cag gaa aag agc ttt gtg caa ttt ctg cag    1059
   Ile Gln Phe Leu Asp Ala Gln Glu Lys Ser Phe Val Gln Phe Leu Gln
       335                 340                 345
   att ggc cag ttt aag gca ttt aaa caa gat gta gac ttc cgc aac tcc    1107
   Ile Gly Gln Phe Lys Ala Phe Lys Gln Asp Val Asp Phe Arg Asn Ser
   350                 355                 360                 365
   aag tca ata aac ttt gtt ggc aac ttt tgg caa gga aac ccg gac ctg    1155
   Lys Ser Ile Asn Phe Val Gly Asn Phe Trp Gln Gly Asn Pro Asp Leu
                   370                 375                 380
   tac gat aag tac gga agg gaa gta aac tat gac gac tcc tac gaa atc    1203
   Tyr Asp Lys Tyr Gly Arg Glu Val Asn Tyr Asp Asp Ser Tyr Glu Ile
               385                 390                 395
   atc gct cgc cgc gtg ctt ggt gct gct cct ccg acc tcc gac aat tac    1251
   Ile Ala Arg Arg Val Leu Gly Ala Ala Pro Pro Thr Ser Asp Asn Tyr
           400                 405                 410
   gaa ttc gtg ccg tct gct ctg gac ttc tac cag act tca ctt cgt gat    1299
   Glu Phe Val Pro Ser Ala Leu Asp Phe Tyr Gln Thr Ser Leu Arg Asp
       415                 420                 425
   ccc gcc ttc tac atg ctc tat aac aag atc atg agc tac att gta cag    1347
   Pro Ala Phe Tyr Met Leu Tyr Asn Lys Ile Met Ser Tyr Ile Val Gln
   430                 435                 440                 445
   tac aag gaa tgg ttg gag ccc tat gat caa gag gta ctt cac tac tcc   1395
   Tyr Lys Glu Trp Leu Glu Pro Tyr Asp Gln Glu Val Leu His Tyr Ser
                   450                 455                 460
   ggt gtc aag atc aat gac gtc agt gtt ggt aac ttg act acc ttc ttc   1443
   Gly Val Lys Ile Asn Asp Val Ser Val Gly Asn Leu Thr Thr Phe Phe
               465                 470                 475
   gag tac tat gac ttc aac gcc acc aat gca gtt ttc tta agt gac caa   1491
   Glu Tyr Tyr Asp Phe Asn Ala Thr Asn Ala Val Phe Leu Ser Asp Gln
           480                 485                 490
   gag att caa caa caa tat tct tca ttc atc gta cgt caa ccg cgt ttg   1539
   Glu Ile Gln Gln Gln Tyr Ser Ser Phe Ile Val Arg Gln Pro Arg Leu
       495                 500                 505
   aac cac gaa cct ttc tcc gtg acc atc gat gtt aag tct gac gtt gag   1587
   Asn His Glu Pro Phe Ser Val Thr Ile Asp Val Lys Ser Asp Val Glu
   510                 515                 520                 525
   gcg gaa gcg tac ttc aag atc ttt gtt ggt cct aaa tat gat gga gaa   1635
   Ala Glu Ala Tyr Phe Lys Ile Phe Val Gly Pro Lys Tyr Asp Gly Glu
                   530                 535                 540
   ggt cgc cct ctt agc ttg gaa gat aac tgg atg aac ttc gtg gaa ttg   1683
   Gly Arg Pro Leu Ser Leu Glu Asp Asn Trp Met Asn Phe Val Glu Leu
               545                 550                 555
   gac tgg ttc acc cac aaa ttg acg tca gga cag aac aag gtt gag cgc   1731
   Asp Trp Phe Thr His Lys Leu Thr Ser Gly Gln Asn Lys Val Glu Arg
           560                 565                 570
   aaa tct gag gaa ttc ttc ttc ttt aaa gag gac tcc gtc tca atg tct   1779
   Lys Ser Glu Glu Phe Phe Phe Phe Lys Glu Asp Ser Val Ser Met Ser
       575                 580                 585
   aag atc tat gaa ctc ctg aaa cag ggc cag gta cct gaa agc atg tcc   1827
   Lys Ile Tyr Glu Leu Leu Lys Gln Gly Gln Val Pro Glu Ser Met Ser
   590                 595                 600                 605
   gaa gac tac gac tct atg cca agc aga ctg atg ttg ccc aga ggc act   1875
   Glu Asp Tyr Asp Ser Met Pro Ser Arg Leu Met Leu Pro Arg Gly Thr
                   610                 615                 620
   ccg ggt ggt ttc cct gta cag ttc ttc gtc ttc gtg tac cca tac caa   1923
   Pro Gly Gly Phe Pro Val Gln Phe Phe Val Phe Val Tyr Pro Tyr Gln
               625                 630                 635
   gct ctc agc aaa gac cta gag gct atg aag aat atc atc ctt gac aac   1971
   Ala Leu Ser Lys Asp Leu Glu Ala Met Lys Asn Ile Ile Leu Asp Asn
           640                 645                 650
   aaa cct ttg ggc tat cca ttt gac cgt cct gtc gag tac ccg tat ctc   2019
   Lys Pro Leu Gly Tyr Pro Phe Asp Arg Pro Val Glu Tyr Pro Tyr Leu
       655                 660                 665
   ttc tta caa cct aat atg tac ttt gaa gac gtc aat atc tac cac aga   2067
   Phe Leu Gln Pro Asn Met Tyr Phe Glu Asp Val Asn Ile Tyr His Arg
   670                 675                 680                 685
   ggc cct caa tac ccc tgg tgg agt aat ggc caa ttc cgt ctg aat gaa   2115
   Gly Pro Gln Tyr Pro Trp Trp Ser Asn Gly Gln Phe Arg Leu Asn Glu
                   690                 695                 700
   gta cct aga caa taaaggagag agaaagagtt cttgaaccaa aacatttaaa       2167
   Val Pro Arg Gln
               705
   gctagtagaa cactatagtc acaataaaat aaaaattttt atagtaaaaa aaaaaaaaaa 2227
   aaa                                                               2230
<210> 6
   <211> 705
   <212> PRT
   <213> Plodia interpunctella
<400> 6
   Met Lys Thr Val Leu Ile Leu Ala Gly Leu Val Ala Leu Ala Ala Gly
    1               5                  10                  15
   Asn Thr Phe Pro Val Phe Arg Tyr Asp His Val Glu Thr Arg Lys Leu
               20                  25                  30
   Glu Gly Asp Leu Leu Gln Tyr Gln Ser Lys Phe Leu Ser Leu Leu Glu
           35                  40                  45
   Asn Val Arg Gln Ile Asp Tyr Glu Ala Glu Tyr Tyr Lys Val Gly Lys
       50                  55                  60
   Gly Tyr Asp Ile Val Ala Ser Ile Glu Asn Tyr Ser Asp Gln Asp Ala
   65                  70                  75                  80
   Val Arg Ala Phe Ala Gly Leu Arg Glu Ile Gly Phe Met Pro Lys Ala
                       85                  90                  95
   Tyr Thr Phe Ser Ile Phe Tyr Asp Arg Gln Arg Glu Glu Ala Lys Ile
                   100                 105                 110
   Ile Tyr Asp Leu Phe Tyr Ser Ala Lys Asp Leu Asp Thr Phe Tyr Lys
               115                 120                 125
   Thr Val Ala Tyr Gly Arg Ile Tyr Phe Asn Glu Tyr Gln Phe Met Tyr
       130                 135                 140
   Ala Phe Tyr Ala Ala Ile Ile Gln Arg Ser Asp Thr Thr Gly Ile Val
   145                 150                 155                 160
   Leu Pro Ala Pro Tyr Glu Leu Tyr Pro Glu Tyr Phe Leu Asn Met Tyr
                       165                 170                 175
   Thr Ile Gln Arg Met Tyr Arg Thr Gln Met Gln Ser Gly Ile Phe Asn
                   180                 185                 190
   Glu Glu Val Ala Ser Asn Tyr Gly Ile Trp Lys Met Asp Asn Asn Tyr
               195                 200                 205
   Tyr Tyr Tyr Tyr Asn Tyr Ser Asn Pro Leu Thr Tyr Arg Asn Gln Glu
       210                 215                 220
   Tyr Arg Leu Ser Tyr Leu Thr Glu Asp Ile Gly Trp Asn Ser Tyr Tyr
   225                 230                 235                 240
   Tyr Tyr Phe His Asn Leu Met Pro Phe Trp Gly Lys Gly Glu Asp Phe
                       245                 250                 255
   Ile Gly Ile Phe Lys Glu Arg Arg Gly Glu Phe Tyr Tyr Tyr Phe Tyr
                   260                 265                 270
   Gln Gln Leu Leu Ser Arg Tyr Tyr Leu Glu Arg Leu Ser Asn Gly Leu
               275                 280                 285
   Gly Glu Ile Pro Asp Phe Ser Trp Tyr Gln Pro Leu Arg Ser Gly Tyr
       290                 295                 300
   Tyr Pro Ala Ile Tyr Thr Ser Ser Ala Tyr Pro Phe Ala Gln Arg Pro
   305                 310                 315                 320
   Asn Tyr Tyr Tyr Met Gly Thr Glu Glu Asn Val Asp Tyr Ile Gln Phe
                       325                 330                 335
   Leu Asp Ala Gln Glu Lys Ser Phe Val Gln Phe Leu Gln Ile Gly Gln
   340 345 350
   Phe Lys Ala Phe Lys Gln Asp Val Asp Phe Arg Asn Ser Lys Ser Ile
   355 360 365
   Asn Phe Val Gly Asn Phe Trp Gln Gly Asn Pro Asp Leu Tyr Asp Lys
   370 375 380
   Tyr Gly Arg Glu Val Asn Tyr Asp Asp Ser Tyr Glu Ile Ile Ala Arg
   385 390 395 400
   Arg Val Leu Gly Ala Ala Pro Pro Thr Ser Asp Asn Tyr Glu Phe Val
   405 410 415
   Pro Ser Ala Leu Asp Phe Tyr Gln Thr Ser Leu Arg Asp Pro Ala Phe
   420 425 430
   Tyr Met Leu Tyr Asn Lys Ile Met Ser Tyr Ile Val Gln Tyr Lys Glu
   435 440 445
   Trp Leu Glu Pro Tyr Asp Gln Glu Val Leu His Tyr Ser Gly Val Lys
   450 455 460
   Ile Asn Asp Val Ser Val Gly Asn Leu Thr Thr Phe Phe Glu Tyr Tyr
   465 470 475 480
   Asp Phe Asn Ala Thr Asn Ala Val Phe Leu Ser Asp Gln Glu Ile Gln
   485 490 495
   Gln Gln Tyr Ser Ser Phe Ile Val Arg Gln Pro Arg Leu Asn His Glu
   500 505 510
   Pro Phe Ser Val Thr Ile Asp Val Lys Ser Asp Val Glu Ala Glu Ala
   515 520 525
   Tyr Phe Lys Ile Phe Val Gly Pro Lys Tyr Asp Gly Glu Gly Arg Pro
   530 535 540
   Leu Ser Leu Glu Asp Asn Trp Met Asn Phe Val Glu Leu Asp Trp Phe
   545 550 555 560
   Thr His Lys Leu Thr Ser Gly Gln Asn Lys Val Glu Arg Lys Ser Glu
   565 570 575
   Glu Phe Phe Phe Phe Lys Glu Asp Ser Val Ser Met Ser Lys Ile Tyr
   580 585 590
   Glu Leu Leu Lys Gln Gly Gln Val Pro Glu Ser Met Ser Glu Asp Tyr
           595                 600                 605
   Asp Ser Met Pro Ser Arg Leu Met Leu Pro Arg Gly Thr Pro Gly Gly
       610                 615                 620
   Phe Pro Val Gln Phe Phe Val Phe Val Tyr Pro Tyr Gln Ala Leu Ser
   625                 630                 635                 640
   Lys Asp Leu Glu Ala Met Lys Asn Ile Ile Leu Asp Asn Lys Pro Leu
                   645                 650                 655
   Gly Tyr Pro Phe Asp Arg Pro Val Glu Tyr Pro Tyr Leu Phe Leu Gln
               660                 665                 670
   Pro Asn Met Tyr Phe Glu Asp Val Asn Ile Tyr His Arg Gly Pro Gln
           675                 680                 685
   Tyr Pro Trp Trp Ser Asn Gly Gln Phe Arg Leu Asn Glu Val Pro Arg
       690                 695                 700
   Gln
   705
<210> 7
   <211> 1076
   <212> DNA
   <213> Plodia interpunctella
<220>
   <221> CDS
   <222> (73)..(834)
<400> 7
   taactgttat tgctcagtga taatagatta gttattatat tgtcaagaag ctgatacgtt 60
   tgcaaaatca tc atg aat ttc gcc ggt aaa gtt gta att gta acc ggt gct 111
                 Met Asn Phe Ala Gly Lys Val Val Ile Val Thr Gly Ala
                   1               5                   10
   agc tcc ggt att gga gca gct aca gct gtg ttc cta tcg aaa cta ggc   159
   Ser Ser Gly Ile Gly Ala Ala Thr Ala Val Phe Leu Ser Lys Leu Gly
        15                  20                  25
   gct aag ctt tct ctg acg gga cgt aac gtc gag aat ctt aag aaa gtt   207
   Ala Lys Leu Ser Leu Thr Gly Arg Asn Val Glu Asn Leu Lys Lys Val
    30                  35                  40                  45
   agt cag gat tgc gaa aaa tcc acc cag aca cac tac atc gcc gcc gac    255
   Ser Gln Asp Cys Glu Lys Ser Thr Gln Thr His Tyr Ile Ala Ala Asp
                       50                  55                  60
   tta acc aaa gaa aaa gat att gaa aat atc gtt aaa agc acc att gat    303
   Leu Thr Lys Glu Lys Asp Ile Glu Asn Ile Val Lys Ser Thr Ile Asp
                   65                  70                  75
   aaa tac ggc caa ctt gac gtc ctg gtc aat aat gct ggc att ctt gag    351
   Lys Tyr Gly Gln Leu Asp Val Leu Val Asn Asn Ala Gly Ile Leu Glu
               80                  85                  90
   act ggt tcc atc gaa aac aca tcg tta gcc cag tac gac agg tta atg    399
   Thr Gly Ser Ile Glu Asn Thr Ser Leu Ala Gln Tyr Asp Arg Leu Met
           95                  100                 105
   aat aca aat gtg cgc tca att tat tac tta acc atg ctg gca gtc cca    447
   Asn Thr Asn Val Arg Ser Ile Tyr Tyr Leu Thr Met Leu Ala Val Pro
   110                 115                 120                 125
   cac ctt ctc aaa acc aaa ggt aac att gtg aat gta tct agt gtc aat    495
   His Leu Leu Lys Thr Lys Gly Asn Ile Val Asn Val Ser Ser Val Asn
                       130                 135                 140
   ggg atc agg tct ttc cct ggt gta ctg gct tac aat gtt tcg aag tca    543
   Gly Ile Arg Ser Phe Pro Gly Val Leu Ala Tyr Asn Val Ser Lys Ser
                   145                 150                 155
   gct gta gat cag ttt aca aga tgt gtt gca ctt gaa ttg gcc ccg aaa    591
   Ala Val Asp Gln Phe Thr Arg Cys Val Ala Leu Glu Leu Ala Pro Lys
               160                 165                 170
   ggg gta cga gtt aat tgt gtg aat cca gga gtc att ttg aca gaa ctg    639
   Gly Val Arg Val Asn Cys Val Asn Pro Gly Val Ile Leu Thr Glu Leu
           175                 180                 185
   cag aag cgt ggg ggt ttg aac gac cag cag tat gca gca ttt ctg gag    687
   Gln Lys Arg Gly Gly Leu Asn Asp Gln Gln Tyr Ala Ala Phe Leu Glu
   190                 195                 200                 205
   aga acc aag gag aca cat gcc ttg ggc cgg ccg gga aaa ccg gag gag    735
   Arg Thr Lys Glu Thr His Ala Leu Gly Arg Pro Gly Lys Pro Glu Glu
                       210                 215                 220
   gtt gca gct act att gct ttc ttg gcc agt gaa tta gca agc aat atc    783
   Val Ala Ala Thr Ile Ala Phe Leu Ala Ser Glu Leu Ala Ser Asn Ile
                   225                 230                 235
   act gga gcc agt gtg cct gta gac ggt ggt cgc cat gcc atg tgt cca   831
   Thr Gly Ala Ser Val Pro Val Asp Gly Gly Arg His Ala Met Cys Pro
           240                 245                 250
   cga taattttttt aataaaatac atgttaattt tttttttact atttacaatt        884
   Arg
   tttcaatcca agcattttac aatgatcaaa gtgtctaaaa ccttttgaat attgtacaat 944
   aaaattttta tatattatag attaagtaaa aacgttcata tacctataat ttgtgtcata 1004
   tggatgtcca tgtgttcata tattttgtta taaccttgtt attttaaaat aaaaacaaat 1064
   aataaaaaaa aa                                                     1076
<210> 8
   <211> 254
   <212> PRT
   <213> Plodia interpunctella
<400> 8
   Met Asn Phe Ala Gly Lys Val Val Ile Val Thr Gly Ala Ser Ser Gly
     1               5                  10                  15
   Ile Gly Ala Ala Thr Ala Val Phe Leu Ser Lys Leu Gly Ala Lys Leu
               20                  25                  30
   Ser Leu Thr Gly Arg Asn Val Glu Asn Leu Lys Lys Val Ser Gln Asp
           35                  40                  45
   Cys Glu Lys Ser Thr Gln Thr His Tyr Ile Ala Ala Asp Leu Thr Lys
       50                  55                  60
   Glu Lys Asp Ile Glu Asn Ile Val Lys Ser Thr Ile Asp Lys Tyr Gly
   65                  70                  75                  80
   Gln Leu Asp Val Leu Val Asn Asn Ala Gly Ile Leu Glu Thr Gly Ser
                   85                  90                  95
   Ile Glu Asn Thr Ser Leu Ala Gln Tyr Asp Arg Leu Met Asn Thr Asn
               100                 105                 110
   Val Arg Ser Ile Tyr Tyr Leu Thr Met Leu Ala Val Pro His Leu Leu
           115                 120                 125
   Lys Thr Lys Gly Asn Ile Val Asn Val Ser Ser Val Asn Gly Ile Arg
   130 135 140
   Ser Phe Pro Gly Val Leu Ala Tyr Asn Val Ser Lys Ser Ala Val Asp
   145 150 155 160
   Gln Phe Thr Arg Cys Val Ala Leu Glu Leu Ala Pro Lys Gly Val Arg
   165 170 175
   Val Asn Cys Val Asn Pro Gly Val Ile Leu Thr Glu Leu Gln Lys Arg
   180 185 190
   Gly Gly Leu Asn Asp Gln Gln Tyr Ala Ala Phe Leu Glu Arg Thr Lys
   195 200 205
   Glu Thr His Ala Leu Gly Arg Pro Gly Lys Pro Glu Glu Val Ala Ala
   210 215 220
   Thr Ile Ala Phe Leu Ala Ser Glu Leu Ala Ser Asn Ile Thr Gly Ala
   225 230 235 240
   Ser Val Pro Val Asp Gly Gly Arg His Ala Met Cys Pro Arg
   245 250

### SEQUENZPROTOKOLL

<110> Duchene, Michael
<120> Rekombinante Allergene aus der Motte Plodia interpunctella
<130> 22034pdemd
<140>
   <141>
<160> 8
<170> PatentIn Ver. 2.1
<210> 1
   <211> 1294
   <212> DNA
   <213> Plodia interpunctella
<220>
   <221> CDS
   <222> (25)..(1089)
<400> 1
   tcaagtgtca gaaaagcagc agca atg gtg gac gcc gct acc ctt gag aaa     51
                              Met Val Asp Ala Ala Thr Leu Glu Lys
                               1               5
   ttg gag gct ggc ttc agc aag ctt gcc gcc tcc gac tca aag tcg ctg    99
   Leu Glu Ala Gly Phe Ser Lys Leu Ala Ala Ser Asp Ser Lys Ser Leu
    10                  15                  20                  25
   ctg aag aaa tac ctc acc agg gag gta ttt gat gct ctc aag aac aag   147
   Leu Lys Lys Tyr Leu Thr Arg Glu Val Phe Asp Ala Leu Lys Asn Lys
                    30                  35                  40
   aag acc tca ttt ggt tca act ctc ctg gat tct atc cag tca ggt gtt   195
   Lys Thr Ser Phe Gly Ser Thr Leu Leu Asp Ser Ile Gln Ser Gly Val
                45                  50                  55
   gag aac tta cat tcg ggt gtt gga att tat gcc cca gat gct gag gca   243
   Glu Asn Leu His Ser Gly Val Gly Ile Tyr Ala Pro Asp Ala Glu Ala
            60                  65                  70
   tat gta gta ttt gca gac ttg ttc gac ccc atc att gaa gat tac cac   291
   Tyr Val Val Phe Ala Asp Leu Phe Asp Pro Ile Ile Glu Asp Tyr His
   75 80 85
   aat ggc ttc aag aaa acc gac aag cac cct ccc aag aac tgg gga gat 339
   Asn Gly Phe Lys Lys Thr Asp Lys His Pro Pro Lys Asn Trp Gly Asp
   90 95 100 105
   gtt gag acc ctc ggg aac ttg gat cct gct ggt gaa ttt gtt gtc tcc 387
   Val Glu Thr Leu Gly Asn Leu Asp Pro Ala Gly Glu Phe Val Val Ser
   110 115 120
   acc cgt gtc cgc tgc ggt cgc tcc atg gaa ggc tac cca ttc aac ccc 435
   Thr Arg Val Arg Cys Gly Arg Ser Met Glu Gly Tyr Pro Phe Asn Pro
   125 130 135
   tgc tta aca gag gcc caa tac aag gaa atg gaa gag aaa gtc tcc tcc 483
   Cys Leu Thr Glu Ala Gln Tyr Lys Glu Met Glu Glu Lys Val Ser Ser
   140 145 150
   aca ctc tcc ggc ctc gag ggt gaa ctg aaa ggc acc ttt ttc cca ctc 531
   Thr Leu Ser Gly Leu Glu Gly Glu Leu Lys Gly Thr Phe Phe Pro Leu
   155 160 165
   aca ggc atg tcc aag gag act caa caa cag ttg att gat gac cac ttc 579
   Thr Gly Met Ser Lys Glu Thr Gln Gln Gln Leu Ile Asp Asp His Phe
   170 175 180 185
   ctg ttc aag gag ggt gat cgc ttc ctc cag gcc gct aac gct tgc cgc 627
   Leu Phe Lys Glu Gly Asp Arg Phe Leu Gln Ala Ala Asn Ala Cys Arg
   190 195 200
   ttc tgg ccc tcc ggt cgt ggc atc tac cac aat gag aac aag act ttc 675
   Phe Trp Pro Ser Gly Arg Gly Ile Tyr His Asn Glu Asn Lys Thr Phe
   205 210 215
   ctg gta tgg tgc aat gag gag gac cac ctc cgt ctg atc tcc atg caa 723
   Leu Val Trp Cys Asn Glu Glu Asp His Leu Arg Leu Ile Ser Met Gln
   220 225 230
   atg ggc ggc gac ctg aag cag gtg tac aag agg ctg gtg agg gga gtg 771
   Met Gly Gly Asp Leu Lys Gln Val Tyr Lys Arg Leu Val Arg Gly Val
   235 240 245
   aac gac atc gcg aag agg atc cca ttc tcg cac aac gag cgg ctg ggc 819
   Asn Asp Ile Ala Lys Arg Ile Pro Phe Ser His Asn Glu Arg Leu Gly
   250 255 260 265
   ttc ctg act ttc tgc ccc acc aac ctg ggc aca acg gtg cgc gca tcg 867
   Phe Leu Thr Phe Cys Pro Thr Asn Leu Gly Thr Thr Val Arg Ala Ser
               270                 275                 280
   gtg cac atc aag ctg ccc aag ctg gcg gcc gac aag gcc aag ctg gag   915
   Val His Ile Lys Leu Pro Lys Leu Ala Ala Asp Lys Ala Lys Leu Glu
               285                 290                 295
   gag gtg gcc agc aag tac cac ctg cag gtg cgc ggc acc cgc ggc gag   963
   Glu Val Ala Ser Lys Tyr His Leu Gln Val Arg Gly Thr Arg Gly Glu
           300                 305                 310
   cac acg gag gcc gag ggc ggc gtc tac gac atc tcc aac aag agg cgc   1011
   His Thr Glu Ala Glu Gly Gly Val Tyr Asp Ile Ser Asn Lys Arg Arg
       315                 320                 325
   atg gga ctc acc gag tac gaa gcc gtc aag gag atg tac gac ggc atc   1059
   Met Gly Leu Thr Glu Tyr Glu Ala Val Lys Glu Met Tyr Asp Gly Ile
   330                 335                 340                 345
   gct gaa ctg atc aaa atc gag aaa tcc ctg taagatgttt aacgatctcg     1109
   Ala Glu Leu Ile Lys Ile Glu Lys Ser Leu
                   350                 355
   cgctatcagt attttttgta ttatttatcg ttttcacata agtattggat gtgaaggggc 1169
   gagggcgaca ctagtcagcg gccttgagcg gggccgggca cgcgggcggc ccactatact 1229
   gtttcgtaaa agtattgtct ataaggaaat ggaaaataaa gacagctagc gttaagacaa 1289
   aaaaa                                                             1294
<210> 2
   <211> 355
   <212> PRT
   <213> Plodia interpunctella
<400> 2
   Met Val Asp Ala Ala Thr Leu Glu Lys Leu Glu Ala Gly Phe Ser Lys
     1               5                  10                  15
   Leu Ala Ala Ser Asp Ser Lys Ser Leu Leu Lys Lys Tyr Leu Thr Arg
               20                  25                  30
   Glu Val Phe Asp Ala Leu Lys Asn Lys Lys Thr Ser Phe Gly Ser Thr
           35                  40                  45
   Leu Leu Asp Ser Ile Gln Ser Gly Val Glu Asn Leu His Ser Gly Val
       50                  55                  60
   Gly Ile Tyr Ala Pro Asp Ala Glu Ala Tyr Val Val Phe Ala Asp Leu
    65                 70                  75                  80
   Phe Asp Pro Ile Ile Glu Asp Tyr His Asn Gly Phe Lys Lys Thr Asp
                   85                  90                  95
   Lys His Pro Pro Lys Asn Trp Gly Asp Val Glu Thr Leu Gly Asn Leu
               100                 105                 110
   Asp Pro Ala Gly Glu Phe Val Val Ser Thr Arg Val Arg Cys Gly Arg
           115                 120                 125
   Ser Met Glu Gly Tyr Pro Phe Asn Pro Cys Leu Thr Glu Ala Gln Tyr
       130                 135                 140
   Lys Glu Met Glu Glu Lys Val Ser Ser Thr Leu Ser Gly Leu Glu Gly
   145                 150                 155                 160
   Glu Leu Lys Gly Thr Phe Phe Pro Leu Thr Gly Met Ser Lys Glu Thr
                   165                 170                 175
   Gln Gln Gln Leu Ile Asp Asp His Phe Leu Phe Lys Glu Gly Asp Arg
               180                 185                 190
   Phe Leu Gln Ala Ala Asn Ala Cys Arg Phe Trp Pro Ser Gly Arg Gly
           195                 200                 205
   Ile Tyr His Asn Glu Asn Lys Thr Phe Leu Val Trp Cys Asn Glu Glu
       210                 215                 220
   Asp His Leu Arg Leu Ile Ser Met Gln Met Gly Gly Asp Leu Lys Gln
   225                 230                 235                 240
   Val Tyr Lys Arg Leu Val Arg Gly Val Asn Asp Ile Ala Lys Arg Ile
                   245                 250                 255
   Pro Phe Ser His Asn Glu Arg Leu Gly Phe Leu Thr Phe Cys Pro Thr
               260                 265                 270
   Asn Leu Gly Thr Thr Val Arg Ala Ser Val His Ile Lys Leu Pro Lys
           275                 280                 285
   Leu Ala Ala Asp Lys Ala Lys Leu Glu Glu Val Ala Ser Lys Tyr His
       290                 295                 300
   Leu Gln Val Arg Gly Thr Arg Gly Glu His Thr Glu Ala Glu Gly Gly
   305                 310                 315                 320
   Val Tyr Asp Ile Ser Asn Lys Arg Arg Met Gly Leu Thr Glu Tyr Glu
                   325                     330                     335
   Ala Val Lys Glu Met Tyr Asp Gly Ile Ala Glu Leu Ile Lys Ile Glu
               340                     345                     350
   Lys Ser Leu
           355
<210> 3
   <211> 1092
   <212> DNA
   <213> Plodia interpunctella
<220>
   <221> CDS
   <222> (31)..(885)
<400> 3
   acaggacagt agacacacaa agccaccacc atg gac gcg atc aag aag aag atg    54
                                    Met Asp Ala Ile Lys Lys Lys Met
                                      1               5
   cag gcg atg aag ctg gag aag gac aac gct ttg gac cgc gct gcc atg    102
   Gln Ala Met Lys Leu Glu Lys Asp Asn Ala Leu Asp Arg Ala Ala Met
        10                  15                  20
   tgc gag cag cag gcc aag gac gcc aac ctc cgt gct gag aag gcc gag    150
   Cys Glu Gln Gln Ala Lys Asp Ala Asn Leu Arg Ala Glu Lys Ala Glu
    25                  30                  35                  40
   gag gag gcc aga caa ttg cag aag aag atc cag acg att gag aac gat    198
   Glu Glu Ala Arg Gln Leu Gln Lys Lys Ile Gln Thr Ile Glu Asn Asp
                    45                  50                  55
   ctg gac cag acg cag gag gcg ctc atg cag gtc aac gcc aag ctg gaa    246
   Leu Asp Gln Thr Gln Glu Ala Leu Met Gln Val Asn Ala Lys Leu Glu
                60                  65                  70
   gag aaa gag aaa gct ctt cag aac gct gag tcc gaa gtc gct gcc ctc    294
   Glu Lys Glu Lys Ala Leu Gln Asn Ala Glu Ser Glu Val Ala Ala Leu
            75                  80                  85
   aac cga cgt atc caa ctg ctg gaa gag gac ctc gag agg tcc gag gag    342
   Asn Arg Arg Ile Gln Leu Leu Glu Glu Asp Leu Glu Arg Ser Glu Glu
           90                  95                  100
   cgc ctc gcc acc gcc aca gcc aaa ctg tcc gaa gcc agc cag gct gcc     390
   Arg Leu Ala Thr Ala Thr Ala Lys Leu Ser Glu Ala Ser Gln Ala Ala
   105                 110                 115                 120
   gat gag tcg gaa cgt gcc cgc aag gtg ctc gag aac agg tca ttg gct     438
   Asp Glu Ser Glu Arg Ala Arg Lys Val Leu Glu Asn Arg Ser Leu Ala
                   125                 130                 135
   gat gaa gag cgt atg gac gct ttg gag aac cag ctg aag gaa gcc agg     486
   Asp Glu Glu Arg Met Asp Ala Leu Glu Asn Gln Leu Lys Glu Ala Arg
               140                 145                 150
   ttc ctt gct gag gaa gcc gac aag aaa tac gat gag gtt gct cgt aag     534
   Phe Leu Ala Glu Glu Ala Asp Lys Lys Tyr Asp Glu Val Ala Arg Lys
           155                 160                 165
   ctg gcc atg gtt gag gct gac ctg gag cgc gcg gag gag cgt gcc gaa     582
   Leu Ala Met Val Glu Ala Asp Leu Glu Arg Ala Glu Glu Arg Ala Glu
       170                 175                 180
   tcc ggc gaa tcc aaa atc gtc gag ctt gag gaa gaa ctg cgc gtg gtt     630
   Ser Gly Glu Ser Lys Ile Val Glu Leu Glu Glu Glu Leu Arg Val Val
   185                 190                 195                 200
   ggc aac aac ttg aaa tcc ctg gaa gtc tcc gag gag aag gcc aac caa     678
   Gly Asn Asn Leu Lys Ser Leu Glu Val Ser Glu Glu Lys Ala Asn Gln
                   205                 210                 215
   cgt gag gag gag tac aaa aat cag atc aaa acc ctc acc acc cgc cta     726
   Arg Glu Glu Glu Tyr Lys Asn Gln Ile Lys Thr Leu Thr Thr Arg Leu
               220                 225                 230
   aag gag gct gag gcc cgc gct gag ttc gcc gag cgt tcc gtg cag aaa     774
   Lys Glu Ala Glu Ala Arg Ala Glu Phe Ala Glu Arg Ser Val Gln Lys
           235                 240                 245
   ctg caa aag gag gtc gac agg ctt gaa gac gaa ctg gtg gct gag aag     822
   Leu Gln Lys Glu Val Asp Arg Leu Glu Asp Glu Leu Val Ala Glu Lys
       250                 255                 260
   gag aaa tac aaa gat att ggt gac gac ctg gac acc ccc ttc gtc gag     870
   Glu Lys Tyr Lys Asp Ile Gly Asp Asp Leu Asp Thr Pro Phe Val Glu
   265                 270                 275                 280
   ctc atc ctc aag gaa taaactcctc acgttggtca cctgggcctg tcccatgcgg     925
   Leu Ile Leu Lys Glu
   285
   ggcagaccca cgggtcattc caagacgcgg ctcttccgcc agcgattcaa catctgtaca 985
   gatgttatat tcattttata ctcatttaaa atatttaaat ctatagtttt atggcggtat 1045
   ttattttcga gtaatataat aaataattta ttacttattt aaaaaaa 1092
<210> 4
   <211> 285
   <212> PRT
   <213> Plodia interpunctella
<400> 4
   Met Asp Ala Ile Lys Lys Lys Met Gln Ala Met Lys Leu Glu Lys Asp
    1               5                  10                  15
   Asn Ala Leu Asp Arg Ala Ala Met Cys Glu Gln Gln Ala Lys Asp Ala
               20                  25                  30
   Asn Leu Arg Ala Glu Lys Ala Glu Glu Glu Ala Arg Gln Leu Gln Lys
           35                  40                  45
   Lys Ile Gln Thr Ile Glu Asn Asp Leu Asp Gln Thr Gln Glu Ala Leu
       50                  55                  60
   Met Gln Val Asn Ala Lys Leu Glu Glu Lys Glu Lys Ala Leu Gln Asn
   65                  70                  75                  80
   Ala Glu Ser Glu Val Ala Ala Leu Asn Arg Arg Ile Gln Leu Leu Glu
                   85                  90                  95
   Glu Asp Leu Glu Arg Ser Glu Glu Arg Leu Ala Thr Ala Thr Ala Lys
               100                 105                 110
   Leu Ser Glu Ala Ser Gln Ala Ala Asp Glu Ser Glu Arg Ala Arg Lys
           115                 120                 125
   Val Leu Glu Asn Arg Ser Leu Ala Asp Glu Glu Arg Met Asp Ala Leu
       130                 135                 140
   Glu Asn Gln Leu Lys Glu Ala Arg Phe Leu Ala Glu Glu Ala Asp Lys
   145                 150                 155                 160
   Lys Tyr Asp Glu Val Ala Arg Lys Leu Ala Met Val Glu Ala Asp Leu
                   165                 170                 175
   Glu Arg Ala Glu Glu Arg Ala Glu Ser Gly Glu Ser Lys Ile Val Glu
               180                 185                 190
   Leu Glu Glu Glu Leu Arg Val Val Gly Asn Asn Leu Lys Ser Leu Glu
           195                 200                 205
   Val Ser Glu Glu Lys Ala Asn Gln Arg Glu Glu Glu Tyr Lys Asn Gln
       210                 215                 220
   Ile Lys Thr Leu Thr Thr Arg Leu Lys Glu Ala Glu Ala Arg Ala Glu
   225                 230                 235                 240
   Phe Ala Glu Arg Ser Val Gln Lys Leu Gln Lys Glu Val Asp Arg Leu
                   245                 250                 255
   Glu Asp Glu Leu Val Ala Glu Lys Glu Lys Tyr Lys Asp Ile Gly Asp
               260                 265                 270
   Asp Leu Asp Thr Pro Phe Val Glu Leu Ile Leu Lys Glu
           275                 280                 285
<210> 5
   <211> 2230
   <212> DNA
   <213> Plodia interpunctella
<220>
   <221> CDS
   <222> (13)..(2127)
<400> 5
   ggtgggtgga cg atg aag act gtc ctg atc tta gct ggc ctc gtg gcc ctg 51
                 Met Lys Thr Val Leu Ile Leu Ala Gly Leu Val Ala Leu
                 1               5                   10
   gcc gcg ggc aac acc ttc ccg gta ttc aga tat gac cac gtc gaa act    99
   Ala Ala Gly Asn Thr Phe Pro Val Phe Arg Tyr Asp His Val Glu Thr
       15                  20                  25
   aga aaa ttg gaa gga gac ctt tta cag tac cag tcg aaa ttt ctg tct   147
   Arg Lys Leu Glu Gly Asp Leu Leu Gln Tyr Gln Ser Lys Phe Leu Ser
   30                  35                  40                  45
   ctt ctt gag aat gtg aga cag att gac tac gaa gcg gag tac tac aaa   195
   Leu Leu Glu Asn Val Arg Gln Ile Asp Tyr Glu Ala Glu Tyr Tyr Lys
                   50                  55                  60
   gtt ggc aag ggt tac gac atc gta gcc agc ata gag aac tat tct gac    243
   Val Gly Lys Gly Tyr Asp Ile Val Ala Ser Ile Glu Asn Tyr Ser Asp
               65                  70                  75
   caa gat gca gtc agg gcg ttt gct ggt ctt cga gaa att ggt ttc atg    291
   Gln Asp Ala Val Arg Ala Phe Ala Gly Leu Arg Glu Ile Gly Phe Met
           80                  85                  90
   ccc aaa gct tac aca ttc tcc att ttc tac gac agg cag aga gaa gaa    339
   Pro Lys Ala Tyr Thr Phe Ser Ile Phe Tyr Asp Arg Gln Arg Glu Glu
       95                  100                 105
   gct aag att att tat gac ttg ttc tac agc gct aaa gat ttg gac act    387
   Ala Lys Ile Ile Tyr Asp Leu Phe Tyr Ser Ala Lys Asp Leu Asp Thr
   110                 115                 120                 125
   ttc tac aag act gta gcc tac ggc cga atc tat ttc aac gag tat cag    435
   Phe Tyr Lys Thr Val Ala Tyr Gly Arg Ile Tyr Phe Asn Glu Tyr Gln
                   130                 135                 140
   ttc atg tat gct ttc tat gct gcg att att cag cgc tct gat acc aca    483
   Phe Met Tyr Ala Phe Tyr Ala Ala Ile Ile Gln Arg Ser Asp Thr Thr
               145                 150                 155
   gga atc gtc tta cca gct cca tat gaa ctg tat cct gaa tat ttc ttg    531
   Gly Ile Val Leu Pro Ala Pro Tyr Glu Leu Tyr Pro Glu Tyr Phe Leu
           160                 165                 170
   aac atg tat acg atc caa aga atg tac cga aca cag atg caa agt ggt    579
   Asn Met Tyr Thr Ile Gln Arg Met Tyr Arg Thr Gln Met Gln Ser Gly
       175                 180                 185
   ata ttc aat gag gaa gtt gct agt aac tat ggt atc tgg aag atg gat    627
   Ile Phe Asn Glu Glu Val Ala Ser Asn Tyr Gly Ile Trp Lys Met Asp
   190                 195                 200                 205
   aat aac tac tat tat tac tac aac tac tct aat ccc ttg acg tac aga    675
   Asn Asn Tyr Tyr Tyr Tyr Tyr Asn Tyr Ser Asn Pro Leu Thr Tyr Arg
                   210                 215                 220
   aat cag gag tac aga ttg tct tat ttg aca gaa gac ata ggc tgg aac    723
   Asn Gln Glu Tyr Arg Leu Ser Tyr Leu Thr Glu Asp Ile Gly Trp Asn
               225                 230                 235
   tct tac tat tac tac ttc cac aat ctt atg cct ttc tgg ggc aaa ggc    771
   Ser Tyr Tyr Tyr Tyr Phe His Asn Leu Met Pro Phe Trp Gly Lys Gly
           240                 245                 250
   gag gac ttt att ggt atc ttc aag gaa cgc cgt gga gaa ttc tac tac    819
   Glu Asp Phe Ile Gly Ile Phe Lys Glu Arg Arg Gly Glu Phe Tyr Tyr
       255                 260                 265
   tac ttc tat cag caa ctc ttg tct cgt tac tac ctt gag cgt ttg agt    867
   Tyr Phe Tyr Gln Gln Leu Leu Ser Arg Tyr Tyr Leu Glu Arg Leu Ser
   270                 275                 280                 285
   aat ggc ttg gga gaa att cca gat ttc tct tgg tac caa cct ctg agg    915
   Asn Gly Leu Gly Glu Ile Pro Asp Phe Ser Trp Tyr Gln Pro Leu Arg
                   290                 295                 300
   agt ggt tac tat cca gct ata tat acg agc tca gcc tat ccg ttt gct    963
   Ser Gly Tyr Tyr Pro Ala Ile Tyr Thr Ser Ser Ala Tyr Pro Phe Ala
               305                 310                 315
   caa cgt ccc aac tat tat tac atg gga act gaa gaa aat gtt gac tac    1011
   Gln Arg Pro Asn Tyr Tyr Tyr Met Gly Thr Glu Glu Asn Val Asp Tyr
           320                 325                 330
   atc caa ttc ctt gat gct cag gaa aag agc ttt gtg caa ttt ctg cag    1059
   Ile Gln Phe Leu Asp Ala Gln Glu Lys Ser Phe Val Gln Phe Leu Gln
       335                 340                 345
   att ggc cag ttt aag gca ttt aaa caa gat gta gac ttc cgc aac tcc    1107
   Ile Gly Gln Phe Lys Ala Phe Lys Gln Asp Val Asp Phe Arg Asn Ser
   350                 355                 360                 365
   aag tca ata aac ttt gtt ggc aac ttt tgg caa gga aac ccg gac ctg    1155
   Lys Ser Ile Asn Phe Val Gly Asn Phe Trp Gln Gly Asn Pro Asp Leu
                   370                 375                 380
   tac gat aag tac gga agg gaa gta aac tat gac gac tcc tac gaa atc    1203
   Tyr Asp Lys Tyr Gly Arg Glu Val Asn Tyr Asp Asp Ser Tyr Glu Ile
               385                 390                 395
   atc gct cgc cgc gtg ctt ggt gct gct cct ccg acc tcc gac aat tac    1251
   Ile Ala Arg Arg Val Leu Gly Ala Ala Pro Pro Thr Ser Asp Asn Tyr
           400                 405                 410
   gaa ttc gtg ccg tct gct ctg gac ttc tac cag act tca ctt cgt gat    1299
   Glu Phe Val Pro Ser Ala Leu Asp Phe Tyr Gln Thr Ser Leu Arg Asp
       415                 420                 425
   ccc gcc ttc tac atg ctc tat aac aag atc atg agc tac att gta cag    1347
   Pro Ala Phe Tyr Met Leu Tyr Asn Lys Ile Met Ser Tyr Ile Val Gln
   430                 435                 440                 445
   tac aag gaa tgg ttg gag ccc tat gat caa gag gta ctt cac tac tcc   1395
   Tyr Lys Glu Trp Leu Glu Pro Tyr Asp Gln Glu Val Leu His Tyr Ser
                   450                 455                 460
   ggt gtc aag atc aat gac gtc agt gtt ggt aac ttg act acc ttc ttc   1443
   Gly Val Lys Ile Asn Asp Val Ser Val Gly Asn Leu Thr Thr Phe Phe
               465                 470                 475
   gag tac tat gac ttc aac gcc acc aat gca gtt ttc tta agt gac caa   1491
   Glu Tyr Tyr Asp Phe Asn Ala Thr Asn Ala Val Phe Leu Ser Asp Gln
           480                 485                 490
   gag att caa caa caa tat tct tca ttc atc gta cgt caa ccg cgt ttg   1539
   Glu Ile Gln Gln Gln Tyr Ser Ser Phe Ile Val Arg Gln Pro Arg Leu
       495                 500                 505
   aac cac gaa cct ttc tcc gtg acc atc gat gtt aag tct gac gtt gag   1587
   Asn His Glu Pro Phe Ser Val Thr Ile Asp Val Lys Ser Asp Val Glu
   510                 515                 520                 525
   gcg gaa gcg tac ttc aag atc ttt gtt ggt cct aaa tat gat gga gaa   1635
   Ala Glu Ala Tyr Phe Lys Ile Phe Val Gly Pro Lys Tyr Asp Gly Glu
                   530                 535                 540
   ggt cgc cct ctt agc ttg gaa gat aac tgg atg aac ttc gtg gaa ttg   1683
   Gly Arg Pro Leu Ser Leu Glu Asp Asn Trp Met Asn Phe Val Glu Leu
               545                 550                 555
   gac tgg ttc acc cac aaa ttg acg tca gga cag aac aag gtt gag cgc   1731
   Asp Trp Phe Thr His Lys Leu Thr Ser Gly Gln Asn Lys Val Glu Arg
           560                 565                 570
   aaa tct gag gaa ttc ttc ttc ttt aaa gag gac tcc gtc tca atg tct   1779
   Lys Ser Glu Glu Phe Phe Phe Phe Lys Glu Asp Ser Val Ser Met Ser
       575                 580                 585
   aag atc tat gaa ctc ctg aaa cag ggc cag gta cct gaa agc atg tcc   1827
   Lys Ile Tyr Glu Leu Leu Lys Gln Gly Gln Val Pro Glu Ser Met Ser
   590                 595                 600                 605
   gaa gac tac gac tct atg cca agc aga ctg atg ttg ccc aga ggc act   1875
   Glu Asp Tyr Asp Ser Met Pro Ser Arg Leu Met Leu Pro Arg Gly Thr
                   610                 615                 620
   ccg ggt ggt ttc cct gta cag ttc ttc gtc ttc gtg tac cca tac caa   1923
   Pro Gly Gly Phe Pro Val Gln Phe Phe Val Phe Val Tyr Pro Tyr Gln
               625                 630                 635
   gct ctc agc aaa gac cta gag gct atg aag aat atc atc ctt gac aac   1971
   Ala Leu Ser Lys Asp Leu Glu Ala Met Lys Asn Ile Ile Leu Asp Asn
           640                 645                 650
   aaa cct ttg ggc tat cca ttt gac cgt cct gtc gag tac ccg tat ctc   2019
   Lys Pro Leu Gly Tyr Pro Phe Asp Arg Pro Val Glu Tyr Pro Tyr Leu
       655                 660                 665
   ttc tta caa cct aat atg tac ttt gaa gac gtc aat atc tac cac aga   2067
   Phe Leu Gln Pro Asn Met Tyr Phe Glu Asp Val Asn Ile Tyr His Arg
   670                 675                 680                 685
   ggc cct caa tac ccc tgg tgg agt aat ggc caa ttc cgt ctg aat gaa   2115
   Gly Pro Gln Tyr Pro Trp Trp Ser Asn Gly Gln Phe Arg Leu Asn Glu
                   690                 695                 700
   gta cct aga caa taaaggagag agaaagagtt cttgaaccaa aacatttaaa       2167
   Val Pro Arg Gln
               705
   gctagtagaa cactatagtc acaataaaat aaaaattttt atagtaaaaa aaaaaaaaaa 2227
   aaa                                                               2230
<210> 6
   <211> 705
   <212> PRT
   <213> Plodia interpunctella
<400> 6
   Met Lys Thr Val Leu Ile Leu Ala Gly Leu Val Ala Leu Ala Ala Gly
     1               5                  10                  15
   Asn Thr Phe Pro Val Phe Arg Tyr Asp His Val Glu Thr Arg Lys Leu
                20                  25                  30
   Glu Gly Asp Leu Leu Gln Tyr Gln Ser Lys Phe Leu Ser Leu Leu Glu
            35                  40                  45
   Asn Val Arg Gln Ile Asp Tyr Glu Ala Glu Tyr Tyr Lys Val Gly Lys
        50                  55                  60
   Gly Tyr Asp Ile Val Ala Ser Ile Glu Asn Tyr Ser Asp Gln Asp Ala
    65                  70                  75                  80
   Val Arg Ala Phe Ala Gly Leu Arg Glu Ile Gly Phe Met Pro Lys Ala
   85 90 95
   Tyr Thr Phe Ser Ile Phe Tyr Asp Arg Gln Arg Glu Glu Ala Lys Ile
   100 105 110
   Ile Tyr Asp Leu Phe Tyr Ser Ala Lys Asp Leu Asp Thr Phe Tyr Lys
   115 120 125
   Thr Val Ala Tyr Gly Arg Ile Tyr Phe Asn Glu Tyr Gln Phe Met Tyr
   130 135 140
   Ala Phe Tyr Ala Ala Ile Ile Gln Arg Ser Asp Thr Thr Gly Ile Val
   145 150 155 160
   Leu Pro Ala Pro Tyr Glu Leu Tyr Pro Glu Tyr Phe Leu Asn Met Tyr
   165 170 175
   Thr Ile Gln Arg Met Tyr Arg Thr Gln Met Gln Ser Gly Ile Phe Asn
   180 185 190
   Glu Glu Val Ala Ser Asn Tyr Gly Ile Trp Lys Met Asp Asn Asn Tyr
   195 200 205
   Tyr Tyr Tyr Tyr Asn Tyr Ser Asn Pro Leu Thr Tyr Arg Asn Gln Glu
   210 215 220
   Tyr Arg Leu Ser Tyr Leu Thr Glu Asp Ile Gly Trp Asn Ser Tyr Tyr
   225 230 235 240
   Tyr Tyr Phe His Asn Leu Met Pro Phe Trp Gly Lys Gly Glu Asp Phe
   245 250 255
   Ile Gly Ile Phe Lys Glu Arg Arg Gly Glu Phe Tyr Tyr Tyr Phe Tyr
   260 265 270
   Gln Gln Leu Leu Ser Arg Tyr Tyr Leu Glu Arg Leu Ser Asn Gly Leu
   275 280 285
   Gly Glu Ile Pro Asp Phe Ser Trp Tyr Gln Pro Leu Arg Ser Gly Tyr
   290 295 300
   Tyr Pro Ala Ile Tyr Thr Ser Ser Ala Tyr Pro Phe Ala Gln Arg Pro
   305 310 315 320
   Asn Tyr Tyr Tyr Met Gly Thr Glu Glu Asn Val Asp Tyr Ile Gln Phe
   325 330 335
   Leu Asp Ala Gln Glu Lys Ser Phe Val Gln Phe Leu Gln Ile Gly Gln
   340 345 350
   Phe Lys Ala Phe Lys Gln Asp Val Asp Phe Arg Asn Ser Lys Ser Ile
   355 360 365
   Asn Phe Val Gly Asn Phe Trp Gln Gly Asn Pro Asp Leu Tyr Asp Lys
   370 375 380
   Tyr Gly Arg Glu Val Asn Tyr Asp Asp Ser Tyr Glu Ile Ile Ala Arg
   385 390 395 400
   Arg Val Leu Gly Ala Ala Pro Pro Thr Ser Asp Asn Tyr Glu Phe Val
   405 410 415
   Pro Ser Ala Leu Asp Phe Tyr Gln Thr Ser Leu Arg Asp Pro Ala Phe
   420 425 430
   Tyr Met Leu Tyr Asn Lys Ile Met Ser Tyr Ile Val Gln Tyr Lys Glu
   435 440 445
   Trp Leu Glu Pro Tyr Asp Gln Glu Val Leu His Tyr Ser Gly Val Lys
   450 455 460
   Ile Asn Asp Val Ser Val Gly Asn Leu Thr Thr Phe Phe Glu Tyr Tyr
   465 470 475 480
   Asp Phe Asn Ala Thr Asn Ala Val Phe Leu Ser Asp Gln Glu Ile Gln
   485 490 495
   Gln Gln Tyr Ser Ser Phe Ile Val Arg Gln Pro Arg Leu Asn His Glu
   500 505 510
   Pro Phe Ser Val Thr Ile Asp Val Lys Ser Asp Val Glu Ala Glu Ala
   515 520 525
   Tyr Phe Lys Ile Phe Val Gly Pro Lys Tyr Asp Gly Glu Gly Arg Pro
   530 535 540
   Leu Ser Leu Glu Asp Asn Trp Met Asn Phe Val Glu Leu Asp Trp Phe
   545 550 555 560
   Thr His Lys Leu Thr Ser Gly Gln Asn Lys Val Glu Arg Lys Ser Glu
   565 570 575
   Glu Phe Phe Phe Phe Lys Glu Asp Ser Val Ser Met Ser Lys Ile Tyr
   580 585 590
   Glu Leu Leu Lys Gln Gly Gln Val Pro Glu Ser Met Ser Glu Asp Tyr
   595 600 605
   Asp Ser Met Pro Ser Arg Leu Met Leu Pro Arg Gly Thr Pro Gly Gly
   610 615 620
   Phe Pro Val Gln Phe Phe Val Phe Val Tyr Pro Tyr Gln Ala Leu Ser
   625 630 635 640
   Lys Asp Leu Glu Ala Met Lys Asn Ile Ile Leu Asp Asn Lys Pro Leu
   645 650 655
   Gly Tyr Pro Phe Asp Arg Pro Val Glu Tyr Pro Tyr Leu Phe Leu Gln
   660 665 670
   Pro Asn Met Tyr Phe Glu Asp Val Asn Ile Tyr His Arg Gly Pro Gln
   675 680 685
   Tyr Pro Trp Trp Ser Asn Gly Gln Phe Arg Leu Asn Glu Val Pro Arg
   690 695 700
   Gln
   705
<210> 7
   <211> 1076
   <212> DNA
   <213> Plodia interpunctella
<220>
   <221> CDS
   <222> (73)..(834)
<400> 7
   taactgttat tgctcagtga taatagatta gttattatat tgtcaagaag ctgatacgtt 60
   tgcaaaatca tc atg aat ttc gcc ggt aaa gtt gta att gta acc ggt gct 111
   Met Asn Phe Ala Gly Lys Val Val Ile Val Thr Gly Ala
   1 5 10
   agc tcc ggt att gga gca gct aca gct gtg ttc cta tcg aaa cta ggc 159
   Ser Ser Gly Ile Gly Ala Ala Thr Ala Val Phe Leu Ser Lys Leu Gly
   15 20 25
   gct aag ctt tct ctg acg gga cgt aac gtc gag aat ctt aag aaa gtt 207
   Ala Lys Leu Ser Leu Thr Gly Arg Asn Val Glu Asn Leu Lys Lys Val
   30 35 40 45
   agt cag gat tgc gaa aaa tcc acc cag aca cac tac atc gcc gcc gac   255
   Ser Gln Asp Cys Glu Lys Ser Thr Gln Thr His Tyr Ile Ala Ala Asp
                   50                  55                  60
   tta acc aaa gaa aaa gat att gaa aat atc gtt aaa agc acc att gat   303
   Leu Thr Lys Glu Lys Asp Ile Glu Asn Ile Val Lys Ser Thr Ile Asp
               65                  70                  75
   aaa tac ggc caa ctt gac gtc ctg gtc aat aat gct ggc att ctt gag   351
   Lys Tyr Gly Gln Leu Asp Val Leu Val Asn Asn Ala Gly Ile Leu Glu
           80                  85                  90
   act ggt tcc atc gaa aac aca tcg tta gcc cag tac gac agg tta atg   399
   Thr Gly Ser Ile Glu Asn Thr Ser Leu Ala Gln Tyr Asp Arg Leu Met
       95                  100                 105
   aat aca aat gtg cgc tca att tat tac tta acc atg ctg gca gtc cca   447
   Asn Thr Asn Val Arg Ser Ile Tyr Tyr Leu Thr Met Leu Ala Val Pro
   110                 115                 120                 125
   cac ctt ctc aaa acc aaa ggt aac att gtg aat gta tct agt gtc aat   495
   His Leu Leu Lys Thr Lys Gly Asn Ile Val Asn Val Ser Ser Val Asn
                   130                 135                 140
   ggg atc agg tct ttc cct ggt gta ctg gct tac aat gtt tcg aag tca   543
   Gly Ile Arg Ser Phe Pro Gly Val Leu Ala Tyr Asn Val Ser Lys Ser
               145                 150                 155
   gct gta gat cag ttt aca aga tgt gtt gca ctt gaa ttg gcc ccg aaa   591
   Ala Val Asp Gln Phe Thr Arg Cys Val Ala Leu Glu Leu Ala Pro Lys
           160                 165                 170
   ggg gta cga gtt aat tgt gtg aat cca gga gtc att ttg aca gaa ctg   639
   Gly Val Arg Val Asn Cys Val Asn Pro Gly Val Ile Leu Thr Glu Leu
       175                 180                 185
   cag aag cgt ggg ggt ttg aac gac cag cag tat gca gca ttt ctg gag   687
   Gln Lys Arg Gly Gly Leu Asn Asp Gln Gln Tyr Ala Ala Phe Leu Glu
   190                 195                 200                 205
   aga acc aag gag aca cat gcc ttg ggc cgg ccg gga aaa ccg gag gag   735
   Arg Thr Lys Glu Thr His Ala Leu Gly Arg Pro Gly Lys Pro Glu Glu
                   210                 215                 220
   gtt gca gct act att gct ttc ttg gcc agt gaa tta gca agc aat atc   783
   Val Ala Ala Thr Ile Ala Phe Leu Ala Ser Glu Leu Ala Ser Asn Ile
               225                 230                 235
   act gga gcc agt gtg cct gta gac ggt ggt cgc cat gcc atg tgt cca   831
   Thr Gly Ala Ser Val Pro Val Asp Gly Gly Arg His Ala Met Cys Pro
           240                 245                 250
   cga taattttttt aataaaatac atgttaattt tttttttact atttacaatt       884
   Arg
   tttcaatcca agcattttac aatgatcaaa gtgtctaaaa ccttttgaat attgtacaat 944
   aaaattttta tatattatag attaagtaaa aacgttcata tacctataat ttgtgtcata 1004
   tggatgtcca tgtgttcata tattttgtta taaccttgtt attttaaaat aaaaacaaat 1064
   aataaaaaaa aa                                                     1076
<210> 8
   <211> 254
   <212> PRT
   <213> Plodia interpunctella
<400> 8
   Met Asn Phe Ala Gly Lys Val Val Ile Val Thr Gly Ala Ser Ser Gly
    1               5                  10                  15
   Ile Gly Ala Ala Thr Ala Val Phe Leu Ser Lys Leu Gly Ala Lys Leu
                20                  25                  30
   Ser Leu Thr Gly Arg Asn Val Glu Asn Leu Lys Lys Val Ser Gln Asp
           35                  40                  45
   Cys Glu Lys Ser Thr Gln Thr His Tyr Ile Ala Ala Asp Leu Thr Lys
       50                  55                  60
   Glu Lys Asp Ile Glu Asn Ile Val Lys Ser Thr Ile Asp Lys Tyr Gly
   65                  70                  75                  80
   Gln Leu Asp Val Leu Val Asn Asn Ala Gly Ile Leu Glu Thr Gly Ser
                   85                  90                  95
   Ile Glu Asn Thr Ser Leu Ala Gln Tyr Asp Arg Leu Met Asn Thr Asn
               100                 105                 110
   Val Arg Ser Ile Tyr Tyr Leu Thr Met Leu Ala Val Pro His Leu Leu
           115                 120                 125
   Lys Thr Lys Gly Asn Ile Val Asn Val Ser Ser Val Asn Gly Ile Arg
           130                 135                 140
   Ser Phe Pro Gly Val Leu Ala Tyr Asn Val Ser Lys Ser Ala Val Asp
   145                 150                 155                 160
   Gln Phe Thr Arg Cys Val Ala Leu Glu Leu Ala Pro Lys Gly Val Arg
                   165                 170                 175
   Val Asn Cys Val Asn Pro Gly Val Ile Leu Thr Glu Leu Gln Lys Arg
               180                 185                 190
   Gly Gly Leu Asn Asp Gln Gln Tyr Ala Ala Phe Leu Glu Arg Thr Lys
           195                 200                 205
   Glu Thr His Ala Leu Gly Arg Pro Gly Lys Pro Glu Glu Val Ala Ala
       210                 215                 220
   Thr Ile Ala Phe Leu Ala Ser Glu Leu Ala Ser Asn Ile Thr Gly Ala
   225                 230                 235                 240
   Ser Val Pro Val Asp Gly Gly Arg His Ala Met Cys Pro Arg
                   245                 250

## Beschreibung

Die vorgestellte Erfindung befaßt sich insbesondere mit dem Problem der allergischen Reaktion auf Invertebratenproteine am Beispiel der Allergie gegen Proteine aus der Dörrobstmotte *Plodia interpunctella.* Sie beschreibt rekombinante Moleküle, die von vier Allergenen dieser Spezies 1abgeleitet sind und ihre Anwendung für Diagnose und Therapie von Allergien und die Detektion von Allergenen in der Umwelt des Menschen.

### Hintergrund der Erfindung

Bis zu 20 % der Bevölkerung der Industriestaaten leiden unter Typ I allergischen Symptomen (Rhinitis, Konjunktivitis, bronchialem Asthma) (Myamoto *et al.*, 1992). Bei der Typ I Allergie bindet das Allergen an IgE-Antikörper auf der Oberfläche von Mastzellen. Das IgE ist an die hochaffinen Fc_{ε}RI-Rezeptoren gebunden, die durch die zusätzliche Bindung der Allergene quervernetzt werden und damit der Mastzelle signalisieren, biologische Mediatoren wie zum Beispiel Histamin freizusetzen (Segal *et al.*, 1977). In den vergangenen Jahren ist gezeigt worden, daß Allergene meist wasserlösliche Proteine sind, die in vielen Fällen in rekombinanter Form erzeugt werden können (Kraft *et al.*, 1999). Noch vor wenigen Jahren wurde ausschließlich speziesspezifische Allegiediagnostik betrieben, bei der Gesamtextrakte natürlicher Allergenquellen, z.B. von Pollen oder Tierhaarextrakte als Antigen eingesetzt wurden. Diese Extrakte sind biochemisch nicht genau definiert, manchmal fehlen wichtige allergene Komponenten. Deshalb wird in den vergangenen Jahren in zunehmender Weise eine komponentenspezifische Diagnose (CRD, "component resolved diagnosis") mit Hilfe von gut definierten, rekombinanten Allergenen eingeführt (Valenta *et al*., 1999).

Während die Allergene außerhalb des Hauses meist mit Pflanzenpollen assoziiert sind, kommen im Haus mehr Allergene aus Tieren vor, sowohl von Schädlingen als auch von Haustieren. Bei den Schädlingen steht als Allergenquelle die Hausstaubmilbe, ein Spinnentier (Thomas und Smith, 1999) an erster Stelle. Besonders in den USA ist die Küchenschabe, ein flügelloses Insekt, auch als Allergenquelle wichtig (Rosenstreich *et al.*, 1997; van Wijnen *et al.,* 1997). Von beiden sind eine Reihe rekombinanter Allergene bekannt (Arruda *et al.*, 1995; Thomas und Smith, 1999). Eine zusätzliche Allergenquelle im Haus sind Schimmelpilze, von denen in den letzten Jahren ebenfalls mehrere allergene Komponenten charakterisiert und für die Diagnostik eingesetzt wurden (Unger *et al.*, 1999).

Diese Erfindung befaßt sich mit einer bisher kaum untersuchten Allergenquelle im häuslichen Bereich, den Motten. Bei den Motten handelt es sich um Insekten, um echte Schmetterlinge (Lepidoptera). Die Hauptvertreter sind *Plodia interpunctella*, die Dörrobstmotte, im englischen Sprachgebrauch "Indian meal moth" und *Tineola bisseliella*, die Kleidermotte, "webbing clothes moth". Die vorliegende Erfindung bezieht sich auf *P. interpunctella*, allerdings sind die verschiedenen Mottenarten nah verwandt und deshalb ist zu erwarten, daß die Allergene der verschiedenen Mottenarten immunologisch kreuzreaktiv sind. Die Dörrobstmotte ist ein Nahrungsmittelparasit, sie wird hauptsächlich in der Küche gefunden und befällt trockene Nahrungsmittel wie Nüsse, Dörrobst, Schokolade, Hafer, Maismehl, Müesli. Es wird vermutet, daß die Dörrobstmotte aus Südamerika stammt. Sie ist der häufiste Nahrungsmittelschädling in den amerikanischen Haushalten und wurde deshalb im Mai 1999 vom Department of Environmental Health & Safety der Harvard Universität zum **"Schädling des Monats" gewählt** (http://www.uos.harvard.edu/ehs/hot_topics/pom_meal_moth.html).Auch in den deutschen Haushalten ist die Dörrobstmotte häufig (zum Beispiel: Vorratsschädling Nr. 1: die Dörrobstmotte. Sendung im Westdeutschen Rundfunk am 9. Mai 1997, von Michael Wiegert-Wegener). Abgestorbene Motten trocknen aus und landen typischerweise über den Hausstaub im Staubsauger. Dieser stößt große Mengen von winzigen Staubpartikeln aus, die auch Proteine der eingesaugten Insekten und damit potentielle Allergene enthalten.

Bisher ist noch von keinem Allergen aus irgendeiner Mottenspezies die Struktur aufgeklärt worden. Außerdem gibt es noch keine Publikation in der gesamten medizinischen Literatur (Medline), die sich mit der Dörrobstmotte im Zusammenhang mit Allergie beschäftigt. Dennoch gibt es eine kleine Zahl von Publikationen, die sich mit Allergien gegen andere Motten beschäftigen. Die Studie von Baldo und Panzani (1988) charakterisiert Extrakte verschiedener Insektenspezies, darunter auch der Kleidermotte (*Tineola bisselliella*) mit IgE Immunoblots, enthält jedoch keine Primärstrukturen. Mehrere Publikationen berichten über allergische Reaktionen gegen Motten oder Seidenraupen bei beruflicher Exposition, zum Beispiel mit Seidenraupen (Komase *et al.* 1997, Suzuki *et al.*, 1995, Wang *et al.,* 1994), verschiedenen Schmetterlingen (Davis and Jenkins 1995), oder Mehlmotten (Storms *et al.*, 1981).

Die vorliegende Anmeldung stellt vier rekombinante Allergene aus der wichtigsten Nahrungsmittelmotte für verschiedene medizinischdiagnostische, umweltanalytische und therapeutische Zwecke zur Verfügung.

Homologe der vier beschriebenen Allergene sind in verschiedenen Spezies in der Vergangenheit bereits untersucht worden, es handelt sich um Argininkinasen, Tropomyosine, Arylphorine und eine Familie von Oxidoreduktasen. Tropomyosine sind als Allergene gut beschrieben (Reese *et al.*, 1999) und auch zum Arylphorin als Allergen bei Schaben (*Periplaneta americana*) gibt es eine Publikation (Wu *et al.*, 1996). In der Literatur sind auch schon einige Redox-Enzyme als Allergen beschrieben, hauptsächlich bei Pilzen und Pflanzen. Das Protein, das zu der gefundenen Oxidoreduktase aus der Motte am nächsten verwandt ist, ist die bakterielle Glukose-1-Dehydrogenase (Nagao *et al.*, 1992), welche selbst nicht als Allergen bekannt ist. Die Argininkinase ist hingegen noch nicht als Allergen identifiziert worden, auch wenn in einer Publikation über ein Allergen Par f 1 aus der Garnele *Parapenaeus fissurus* Peptidsequenzen veröffentlicht wurden, die Sequenzähnlichkeiten zu Argininkinasen anderer Spezies aufweisen (Lin *et al.*, 1993). Diese Ähnlichkeiten wurden jedoch in der Veröffentlichung nicht beschrieben. Die Argininkinase ist ein Enzym, das in Muskeln von Invertebraten Argininphosphat als Energie-Reservestoff bildet (Wyss *et al.*, 1995). Auch bei Insekten wurde die Argininkinase in ihrer Primärstruktur aufgeklärt (Kucharski und Maleszka, 1998), allerdings nie als Allergen beschrieben.

Die vorliegende Erfindung basiert auf der Erkenntnis, daß die Dörrobstmotte, die in unseren Wohnungen sehr häufig als Nahrungsmittelschädling auftritt, auch eine Allergenquelle darstellen kann. Etwa die Hälfte der untersuchten Patientenseren wiesen IgE gegen Mottenallergene auf. Die Anmeldung stellt molekular genau definierte Reagenzien zur Verfügung, die von den beschriebenen Allergen p40 (Argininkinase), p33 (Tropomyosin), p84 (Arylphorin) und p27 (Oxidoreduktase) abgeleitet sind und einerseits eine exakt definierte und einfache *in vitro* und *in vivo* Diagnose und Therapie der Allergie gegen Motten ermöglichen, andererseits den Nachweis von Mottenproteinen in Proben aus Haushalt, Schule oder Betrieb. Die Bezeichnungen der Allergene erfolgen in Anlehnung an ihre Molekulargewichte in kDa.

Das Allergen p40 ist überdies ein neues Panallergen von wirbellosen Tieren, das auch in der Hausstaubmilbe, in der Schabe und in Meeresfrüchten gefunden wird und in diesen Spezies immunologisch verwandt mit p40 aus der Motte ist. So ist es denkbar, daß man sich durch den Kontakt mit Motten oder Milben sensibilisiert und in der Folge eine Nahrungsmittelallergie gegen Meeresfrüchte entwickelt. Für die Untersuchung einer solchen Kreuzsensibilisierung können das rekombinante p40 oder nahe verwandte Moleküle eingesetzt werden.

### Beschreibung der Erfindung

Die Erfindung betrifft eine Nukleinsäure, kodierend für ein allergenes Polypeptid aus Anthropoden, umfassend
(a) die in SEQ ID No. 1 dargestellte Sequenz,
(b) eine von einer Sequenz gemäß (a) auf Grund einer Degeneration des genetischen Codes abweichende Sequenz, oder/und
(c) eine Sequenz mit einer Identität > 80 % zu einer der Sequenzen unter (a) und/oder (b),
sowie eine Nukleinsäure, umfassend einen Bereich, der für ein Polypeptid mit der in SEQ ID No. 2 dargestellten Sequenz kodiert.

Ein erster Aspekt der Erfindung sind rekombinante DNA-Moleküle, die Nukleotidsequenzen (I) aufweisen, die Polypeptide kodieren, die die Antigenität des Allergens p40 besitzen und aus Arthropoden isoliert sind. Die rekombinanten DNA-Moleküle umfassen auch degenerierte Varianten dieser Nukleotidsequenzen.

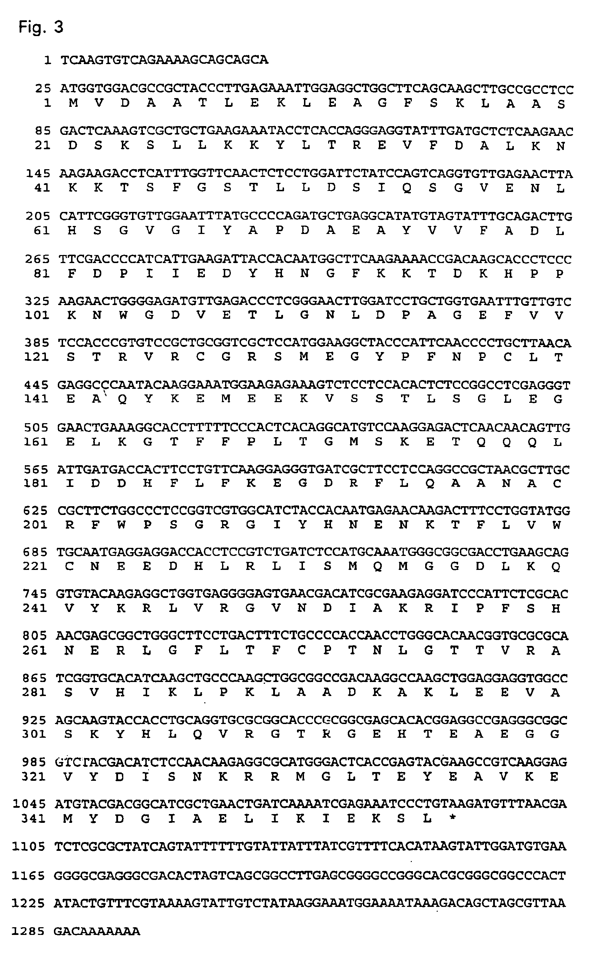

Die rekombinanten DNA-Moleküle können auch Nukleotidsequenzen enthalten, die für Polypeptide kodieren, die antigene Kreuzreaktivität und einen hohen Grad von Identität (vorzugsweise > 50 %, insbesondere > 60 % oder > 75 %) mit dem Allergen p40 aus Arthropoden besitzen, das in der Abbildung 3 angegeben ist. Die Bezeichnung p40 bezieht sich auf das Molekulargewicht des Polypeptids in kDa.

Der Ausdruck "Hybridisierung unter hochstringenten Bedingungen" gemäß der vorliegenden Erfindung wird wie bei Sambrook et al. (Molecular Cloning: A Laboratory Manual, Cold Spring Harbor Laboratory Press (1989)1.101-1.104) verwendet. Bevorzugt liegt eine hochstringente Hybridisierung gemäß der vorliegenden Erfindung vor, wenn nach Waschen für 1 Stunde mit 1 x SSC und 0,1 % SDS bei 50 °C, bevorzugt bei 55 °C, mehr bevorzugt bei 62 °C und am meisten bevorzugt bei 68 °C, und mehr bevorzugt für 1 Stunde bei 0,2 x SSC und 0,1 % SDS bei 50 °C, bevorzugt bei 55 °C, mehr bevorzugt bei 62 °C und am meisten bevorzugt bei 68 °C noch ein positives Hybridisierungssignal beobachtet werden kann.

Der Ausdruck "Identität", wie hierin verwendet, kann durch die Gleichung I(%) = [1-V/X] x 100 definiert werden, worin I die Identität in % ausgedrückt bedeutet, X die Gesamtzahl der Nukleobasen einer Nukleotidsequenz für p40 ist und V die Anzahl an davon abweichenden Nukleobasen der zu vergleichenden Sequenzen ist.

Ein zweiter Aspekt der Erfindung sind rekombinante Expressionsvektoren, die eine Expressionskontrollsequenz aufweisen, die operativ mit einem der oben beschriebenen Moleküle verknüpft ist.

Ein dritter Aspekt der Erfindung ist eine Wirtszelle, die mit einem Vektor nach dem zweiten Aspekt der Erfindung transformiert ist.

Ein vierter Aspekt der Erfindung ist ein rekombinantes oder synthetisches Protein oder Polypeptid, das antigene Epitope des p40 Moleküls besitzt, die in der Aminosäuresequenz von Abbildung 3 enthalten sind. Das Protein oder Polypeptid kann dabei mit einem weiteren heterologen Polypeptid wie einer zellulosebindenden Domäne, β-Galaktosidase oder Glutathion-S-Transferase oder irgendeinem anderen Polypeptid fusioniert sein, das in prokaryontischen oder eukaryontischen Zellen exprimiert werden kann. Das Protein oder Polypeptid, das mit p40 kreuzreaktiv ist, kann dabei mit analytisch nachweisbaren Gruppen oder mit wasserlöslichen oder wasserunlöslichen Phasen konjugiert sein, die für die Durchführung des Nachweises von Antikörpern wie zum Beispiel IgA, IgD, IgE, IgG oder IgM geeignet sind. In den Aspekten der Erfindung, die sich mit in vitro Diagnostik befassen (siehe unten), können die Peptide der Erfindung a) an eine wasserunlösliche Phase durch physikalische Adsorption oder eine kovalente Bindung gekoppelt sein oder b) kovalent an eine analytisch nachweisbare Gruppe (Markierung) gekoppelt sein.

Die erfindungsgemäßen Polypeptide können als Immunogene zur Herstellung von Antikörpern eingesetzt werden. Zur Herstellung von für die allergenen Determinanten spezifischen Antikörpern können Standardprotokolle herangezogen werden. Die Antikörper können dann z.B. zum Nachweis von Allergenen und/oder zur Therapie verwendet werden.

Die Erfindung umfaßt weiterhin eine Zusammensetzung zur Verwendung als Arzneimittel, umfassend eine Nukleinsäure, einen Vektor, eine Zelle, ein Polypeptid oder einen Antikörper, wie hierin definiert, als Wirkstoff. Die Zusammensetzung kann pharmazeutisch annehmbare Hilfsstoffe sowie ggf. weitere Wirkstoffe enthalten. Die Zusammensetzung kann für diagnostische oder/und therapeutische Zwecke verwendet werden, insbesondere für Therapie oder/und Diagnose von allergischen Erkrankungen.

Der fünfte Aspekt der Erfindung ist eine in vitro Methode zur Diagnose von Allergie gegen Arthropodenproteine, die die humoralen Antikörper bestimmt, die gegen die Arthropodenproteine gerichtet sind. Die umfaßten Allergien sind meistens gegen Insekten gerichtet. Die relevanten Antikörper sind meistens von der IgE Klasse, aber auch IgG-Antikörper können wichtige Information über die Allergie liefern. Im Normalfall umfaßt diese Methode den Kontakt einer Körperflüssigkeit aus einem Patienten mit einem Polypeptid der Erfindung. Die Mengenverhältnisse und Bedingungen werden so gewählt, daß sich Immunkomplexe zwischen dem Polypeptid und Antikörpern in der Probe in einer Menge ausbilden, die eine Funktion der Menge der Antikörper in der Probe ist. Der Immunkomplex wird dann mit einer der an sich bekannten Methoden gemessen. Etwas spezifischer ausgedrückt, eine bevorzugte Methode des fünften Aspekts der Erfindung besteht darin, eine Probe einer Körperflüssigkeit, die zum Beispiel IgE Antikörper enthält, mit einem Polypeptid der Erfindung und einem Anti-IgE-Antikörper in Kontakt zu bringen, so daß sich ein lgE-Polypeptid-Anti-lgE-Immunkomplex bildet. Im Normalfall ist entweder das Polypeptid oder der Anti-lgE-Antikörper an eine feste Phase gekoppelt, die entweder unlöslich ist, oder im Testpuffer gefällt werden kann, so daß der Immunkomplex von dem Testpuffer getrennt werden kann. Der Detektionsschritt kann in diesen Varianten unter Verwendung einer analytisch nachweisbaren Gruppe (Markierung) ausgeführt werden, die entweder kovalent an den IgE-Antikörper gekoppelt ist (in diesem Fall ist das Polypeptid an die Festphase gekoppelt) oder an das Polypeptid (in diesem Fall ist der Anti-IgE-Antikörper an die Festphase gekoppelt). Wenn IgG-Antikörper bestimmt werden sollen, dann wird der Anti-IgE-Antikörper durch einen Anti-lgG-Antikörper ersetzt.

Ein sechster Aspekt der Erfindung ist eine in vitro Methode, die eine zelluläre Immunreaktion, auf das Polypeptid der Erfindung mißt und ein rekombinantes oder synthetisches Polypeptid wie im vierten Aspekt beschrieben verwendet, um die zelluläre Immunreaktion, zu stimulieren. Als zelluläre Reaktionen können die Histaminfreisetzung aus basophilen Granulozyten oder die Proliferation von T-Lymphozyten, gemessen durch Aufnahme von ³H-Thymidin gemessen werden, ebenso die Stimulation von eosinophilen Granulozyten, gemessen durch die Freisetzung von Mediatoren, wie zum Beispiel dem eosinophilen kationischen Protein. Die Proben, die in den oben beschrieben Methoden verwendet werden, sind meistens aus Blut gewonnen, wie zum Beispiel heparinisiertem Vollblut, Serum oder Plasma.

Ein siebenter Aspekt der Erfindung betrifft nur das p40 Allergen und besteht darin, durch Messung der Enzymaktivität des p40 Allergens und seiner Homologen, der Argininkinaseaktivität (EC 2.7.3.3), das Vorhandensein von Arthropodenallergenen in Proben aus der Umwelt des Menschen zu messen, beispielsweise in Staubproben aus Haushalt oder Betrieben. Die Argininkinase katalysiert die reversible Umwandlung von L-Arginin und Adenosintriphosphat (ATP) in N-Phospho-L-Arginin und Adenosindiphosphat (ADP). Für die Messung der Argininkinaseaktivität sind in der Literatur Standardmethoden beschrieben, die zum Beispiel das entstehende Produkt ADP indirekt messen (Anisike *et al*., 1975).

Der achte Aspekt der Erfindung besteht darin, mit Hilfe eines Immunoassays das Vorhandensein des p40 Allergens oder seiner Homologen in Proben aus der Umwelt des Menschen zu messen, beispielsweise in Staubproben aus Haushalt oder Betrieben. Der Immunassay besteht darin, daß man einen monoklonalen Antikörper aus der Maus, der nach Standardmethoden gegen eines der Polypeptide der Erfindung gewonnen wird, oder ein Antiserum aus einem Wirbeltier, wie zum Beispiel, Kaninchen, Ziege, Schaf, Huhn, das gegen eines der

Polypeptide der Erfindung gerichtet ist, mit der Umweltprobe in Kontakt bringt, die auf das p40 Allergen oder seine Homologen mit einer Sequenzidentität >70% hierzu getestet werden soll. Dabei ist der erste Antikörper oder das Antiserum typischerweise kovalent oder nichtkovalent an eine feste Phase gekoppelt, die Umweltprobe wird in wässriger Lösung oder in einem polaren Lösungsmittel gelöst angeboten. Nach einem Waschschritt wird das gebundene p40 Allergen oder seine Homologen mit einem zweiten, markierten monoklonalen Antikörper oder einem Antiserum detektiert.

Bei diesem Verfahren kann insbesondere ein p40-Homologes aus einer beliebigen Spezies, besonders bevorzugt aus Motte oder Milbe, am meisten bevorzugt aus Hausstaubmilbe, bestimmt werden.

Der neunte Aspekt der Erfindung besteht darin, aus dem synthetischen oder rekombinanten Polypeptid der Erfindung ein Arzneimittel herzustellen, das zur Hyposensibilisierung (Immunotherapie) von Patienten mit Allergie gegen p40 oder seine Homologen mit einer Sequenzidentität > 70% hierzu eingesetzt werden kann.

Die Anmeldung offenbart ferner die Herstellung solcher Fragmente oder Teilpeptide oder Multimere des Polypeptids der Erfindung, die zwar ein oder mehrere Epitope, insbesondere IgE, IgG oder/und IgA-Epitope, des p40 Allergens oder seiner Homologen enthalten, aber nicht oder nur in einem stark eingeschränkten Maß zu einer anaphylaktischen Reaktion führen können. Multimere eines Allergens wirken oftmals weniger anaphylaktisch als Monomere. IgG und IgA-Epitope können eine geringere anaphylaktische Wirkung als IgE-Epitope aufweisen. Diese Derivate der Polypeptide der Erfindung können zu einem Arzneimittel entwickelt werden, das entweder zur passiven Therapie des Effektororgans eingesetzt werden (Nase, Conjunctiva, Lunge), um einer Freisetzung von Mediatoren bei einer späteren Allergenexposition vorzubeugen, oder ebenfalls zu einer aktiven Immunotherapie im Sinne einer Hyposensibilisierung.

Die Anmeldung offenbart ferner ein diagnostisches Mittel zum Nachweis einer Allergie bei einem Patienten, wobei dieses Mittel ein Polypeptid oder einen Antikörper wie oben beschrieben, enthält.

Mit den Erkentnissen der vorliegenden Erfindung ist es möglich, eine speziesspezifische Allergiediagnostik unter Verwendung einer Motte, insbesondere der Dörrobstmotte zu betreiben. Hierzu können die Dörrobstmotte, Extrakte davon, wie etwa Gesamtextrakte oder einzelne Bestandteile, insbesondere in Form von Teilextrakten zur Bestimmung einer allergischen Reaktion, beispielsweise als Antigen eingesetzt werden.

Daneben ist es auch möglich, eine komponentenspezifische Allergiediagnostik durchzuführen, in dem Proben auf die einzelnen, oben beschriebenen Allergene untersucht werden. In diesem Zusammenhang ist die Diagnose einer Argininkinase, insbesondere aus einer Motte oder aus einer Milbe, beispielsweise der Hausstaubmilbe, von besonders großem Interesse. Aber auch die anderen identifizierten Allergene sowie deren Homologen aus Arthropoden können für eine komponentenspezifische Allergiediagnostik herangezogen werden.

Auf Grund der hierin präsentierten Ergebnisse kann eine Argininkinase zur Herstellung eines Arzneimittels oder/und eines diagnostischen Mittels zur Behandlung von allergischen Erkrankungen oder/und zur Bestimmung von Allergenen verwendet werden. Bevorzugt wird hierzu eine Argininkinase aus einem Arthropoden, insbesondere aus Motte oder aus Milbe, z.B. Hausstaubmilbe eingesetzt bzw. ein Test auf das Vorhandensein einer solchen Argininkinase durchgeführt. Bei der Argininkinase handelt es sich um p40 oder eine Argininkinase, die zu p40 eine Identität von > 70 %, bevorzugt > 80 %, aufweist und bevorzugt mit p40 konzentriert.

Grundsätzlich eröffnet sich somit eine breite Verwendung der erfindungsgemäß gefundenen Allergen und der dafür kodierenden Nukleinsäuren auf medizinisch-diagnostischem, umweltanalytischem und therapeutischem Gebiet.

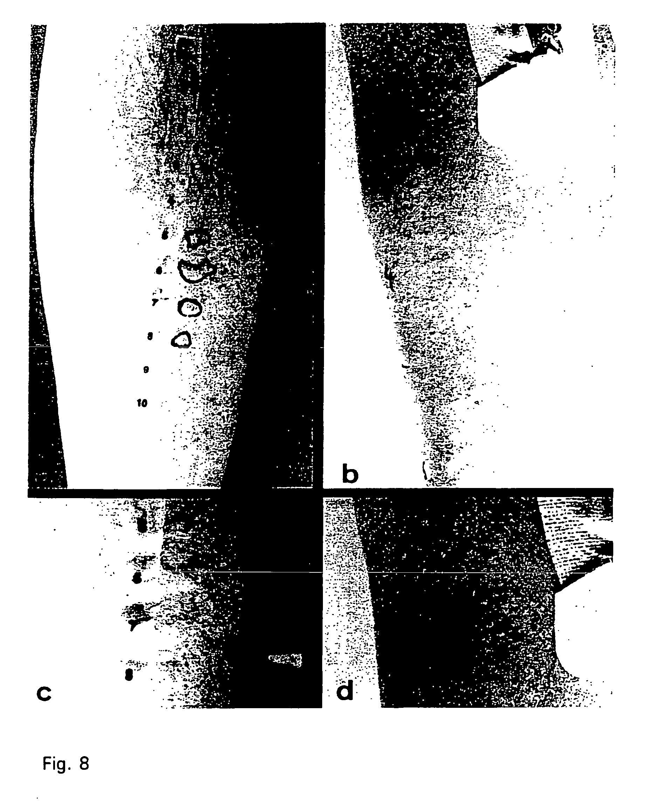

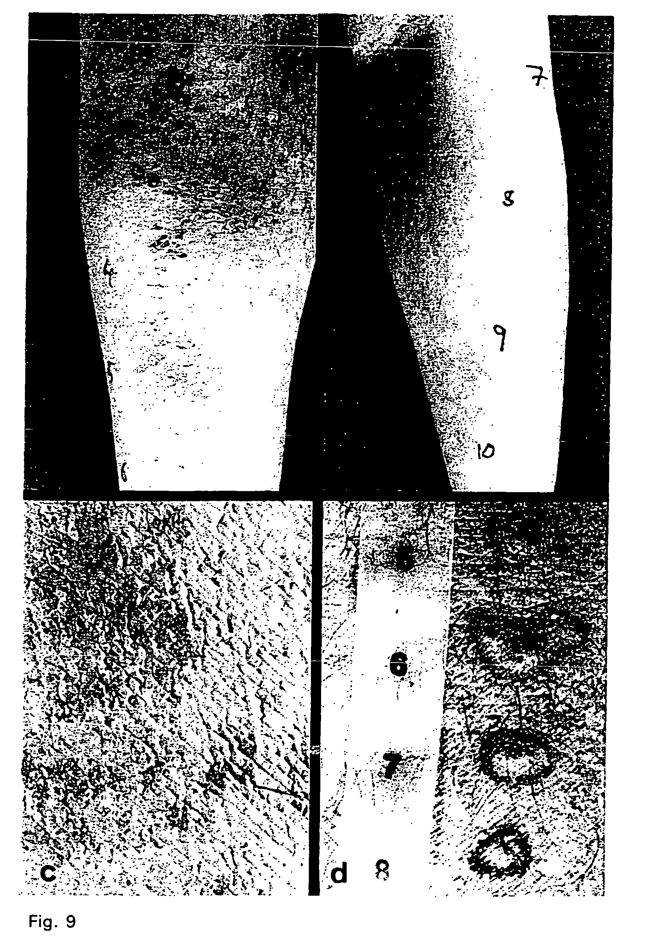

Die Erfindung wird durch die folgenden Beispiele und die beigefügten Figuren weiter erläutert. Die Figuren zeigen:
Fig. 1:
   Immunoblotstreifchen mit Gesamtextrakt aus Larven der Dörrobstmotte, untersucht auf IgE in den Seren von 90 Patienten mit allergischen Beschwerden in Innenräumen (H1 - H90, jeweils oberer Teil). Im jeweils unteren Teil wurden Immunoblotstreifchen mit rekombinantem p40 Allergen mit Hexahistidintag mit denselben Seren geprobt. Die Positionen von Molgewichtsmarkern sind auf der linken Seite in kDa angegeben. K ist die Pufferkontrolle ohne Zugabe von Serum.
Fig. 2:
   Immunoblotstreifchen mit Gesamtextrakt aus Larven der Dörrobstmotte (jeweils oberer Teil), untersucht auf IgE in den Seren von Patienten mit allergischen Beschwerden in Innenräumen plus atopischer Dermatitis (AH1 - AH12), von Patienten mit Pollenallergie ohne angegebene Beschwerden im Haus (P1 - P20) und von Normalpersonen (N1 - N10). Im jeweils unteren Teil wurden Immunoblotstreifchen mit rekombinantem p40 Allergen mit Hexahistidintag mit denselben Seren untersucht. Die Positionen von Molgewichtsmarkern sind auf der linken Seite in kDa angegeben. K ist die Pufferkontrolle ohne die Zugabe von Serum.
Fig.3:
   cDNA (SEQ ID No. 1) und davon abgeleitete Proteinsequenz (SEQ ID No. 2) des Allergens p40 aus *Plodia interpunctella*
Fig. 4:
   cDNA (SEQ ID No. 3) und davon abgeleitete Proteinsequenz (SEQ ID No. 4) des Allergens p33 aus *Plodia interpunctella*
Fig. 5:
   cDNA (SEQ ID No. 5) und davon abgeleitete Proteinsequenz (SEQ ID No. 6) des Allergens p84 aus *Plodia interpunctella*
Fig. 6:
   cDNA (SEQ ID No. 7) und davon abgeleitete Proteinsequenz (SEQ ID No. 8) des Allergens p27 aus *Piodia interpunctella*
Fig. 7:
   IgE-Immunoblot. Streifchen mit rekombinantem p40 Fusionsprotein mit einer Zellulose-bindenden Domäne wurden mit einer Auswahl der oben beschriebenen Seren getestet. Auf der rechten Seite sind die Molekulargewichtsmarker angegeben
Fig. 8:
   Soforttypreaktionen beim Hauttest mit dem rekombinanten p40 Allergen mit Hexahistidintag.
   a: Pricktest bei dem mottenallergischen Patienten AH11. Keine Hautreaktivität auf Konzentrationen Nr. 10 und 9. Qaddeln und Rötung bei den Konzentrationen Nr. 8 (3.12 ng/µl) bis Nr. 5 (25 ng/µl). Die höheren Konzentrationen wurden nicht mehr getestet. + + Positivkontrolle (Histamindihydrochlorid), - Negativkontrolle (0,9 % NaCl). Die Quaddeln sind mit einem Stift markiert.
   b: Reibetest am kontralateralen Unterarm desselben Patienten, starke Quaddelbildung und Hautrötung in den Konzentrationen Nr. 2 (200 ng/µl) und Nr. 3 (100 ng/µl).
   c: Vergrößerung des Bereichs von Fig. 8a bevor die Quaddeln angezeichnet wurden. Die urtikarielle Reaktion mit der Bildung von Pseudopodien (Nr. 6) ist gut zu erkennen.
   d: Vergrößerung von Fig. 8b: Quaddelbildung im Reibetest bei der Konzentration Nr. 2 nach 20 min.
Fig. 9:
   Spätphasenreaktionen nach 24 h bei der Hauttestung mit dem rekombinanten p40 Allergen mit Hexahistidintag.
   a: Reibetest: ekzematöse Reaktion in den Konzentrationen Nr. 6 (12.5 ng/µl) bis Nr. 2 (200 ng/µl). Nr. 1 wurde nicht durchgeführt.
   b: Reibetest: keine ekzematöse Reaktionen in den Konzentrationen Nr. 10 bis Nr. 7.
   c: Vergrößerung von Fig. 9a: Ekzematöse Reaktion bei Konzentration Nr. 4.
   d: Pricktest: Infiltrierte Papeln innerhalb der markierten Grenzen der vorangegangenen Soforttyp-Reaktion.
Fig. 10:
   Immunoblot-Inhibitionsexperiment. Drei mit rekombinantem p40 Allergen aus der Dörrobstmotte positive Seren wurden verwendet, um mit und ohne Präinkubation mit rekombinantem p40 allergenhaltige Extrakte aus verschiedenen Spezies (Dörrobstmotte, Küchenschabe, Hausstaubmilbe, Hummer, Garnele, Miesmuschel und Kabeljau) zu testen. Die Molekulargewichte sind auf der linken Seite der Immunoblots angegeben, von oben nach unten 66, 46, 30 und 21 kDa.

SEQ ID No. 1 zeigt die cDNA des Allergens p40 aus *Plodia interpunctella*,
SEQ ID No. 2 zeigt die davon abgeleitete Proteinsequenz,
SEQ ID No. 3 zeigt die cDNA des Allergens p33 aus *Plodia interpunctella*,
SEQ ID No. 4 zeigt die davon abgeleitete Proteinsequenz,
SEQ ID No. 5 zeigt die cDNA des Allergens p84 aus *Plodia interpunctella,*
SEQ ID No. 6 zeigt die davon abgeleitete Proteinsequenz,
SEQ ID No. 7 zeigt die cDNA des Allergens p27 aus *Plodia interpunctella*, und
SEQ ID No. 8 zeigt die davon abgeleitete Proteinsequenz.

### Beispiele:

### Beispiel 1:

### Test von verschiedenen Gruppen von Allergikern und Normalpersonen auf IgE-Antikörper gegen Mottenantigene aus Larven der Dörrobstmotte P. interpunctella.

Da im klinischen Bereich eine mögliche Allergie gegen Motten bislang kaum Beachtung gefunden hat, konnte bei der Auswahl der Patienten keine Gruppe definiert werden, die klinische Beschwerden nach Kontakt mit Mottenallergenen als Symptom angab. Deshalb stellten wir für unsere Arbeit die folgenden Gruppen zusammen, die auf IgE-Antikörper gegen Mottenproteine getestet wurden:
1. Patienten mit Typ I allergischen Beschwerden (Rhinitis, Conjunctivitis, allergisches Asthma bronchiale) in Innenräumen (n = 90, Patienten H1-H90),
2. Patienten mit atopischer Dermatitis und Typ I allergischen Beschwerden (Rhinitis, Conjunctivitis, allergisches Asthma bronchiale) in Innenräumen (n = 12, Patienten AH 1 -AH 12),
3. Patienten mit nachgewiesener Pollenallergie ohne Typ I allergische Beschwerden (Rhinitis, Conjunctivitis, allergisches Asthma bronchiale) in Innenräumen (n = 20, Patienten P1 - P20),
4. Probanden ohne atopische Dermatitis und ohne nachgewiesene Typ I Allergien (n=10, Probanden N1 - N10).

### IgE-Reaktivität von natürlichen Mottenextrakten

Präparationen von zwei verschiedenen Mottenspezies wurden verwendet, um mottenspezifische IgE-Antikörper in Patientenseren zu detektieren. Die eine Präparation ist ein kommerziell erhältliches Homogenisat von Faltern der Mehlmotte *Ephestia kuehniella* (Allergon, Pharmacia Upjohn, Uppsala, Schweden). Die andere Präparation wurde aus Mottenlarven (Wanderstadium, kurz vor der Verpuppung) von der Dörrobstmotte *Plodia interpunctella* hergestellt. Die Insektenproben (5 Larven) wurden in 0,2 ml PBS homogenisiert, im Verhältnis von 1:1 mit Laemmli-Auftragspuffer versetzt und auf einem 12,5 % SDS-Polyacrylamidgel aufgetrennt (Fling und Gregerson, 1986). Es wurde ein präparatives Gel verwendet, auf das etwa 20 µg Gesamtprotein pro cm aufgetragen wurden. Als Marker diente ein Rainbow-Marker (Amersham Pharmacia). Das Gel wurde nach der Elektrophorese auf Nitrozellulose (Schleicher & Schuell, Dassel, Deutschland) geblottet (Towbin *et al.*, 1979) und in 0,5 cm Streifen geschnitten.

Der Test der Patientenseren auf IgE gegen Motten wurde analog zu der von Jarolim *et al.* (1989) beschrieben Methode durchgeführt. Die Streifen wurden 2 x 5 min und 1 x 30 min in Puffer G (42 mM Na₂HPO₄, 6,4 mM NaH₂PO₄, 0,5 % (v/v) Tween 20, 0,5 % (w/v) Rinderserumalbumin, 0,05 % (w/v) NaN₃, pH 7.5) bei Raumtemperatur abgesättigt, dann in 1 ml Volumen in 1:10 (wenn nicht anders beschrieben) mit Puffer G verdünnten Patientenseren über Nacht bei 4 °C gekippt. Die Streifen wurden 2 x 5 min und 1 x 30 min bei Raumtemperatur in Puffer G gewaschen, dann über Nacht mit einer 1:10 Verdünnung eines ¹²⁵I-markierten Anti-Human-lgE Antikörpers (Amersham Pharmacia) bei Raumtemperatur gekippt, wie oben gewaschen, getrocknet und aufgeklebt. Gebundenes mottenspezifisches IgE wurde so mit dem radioaktiv markierten Anti-IgE-Antikörper detektiert und die positiven Signale wurden mittels Autoradiographie auf einem Röntgenfilm (Kodak) sichtbar gemacht.

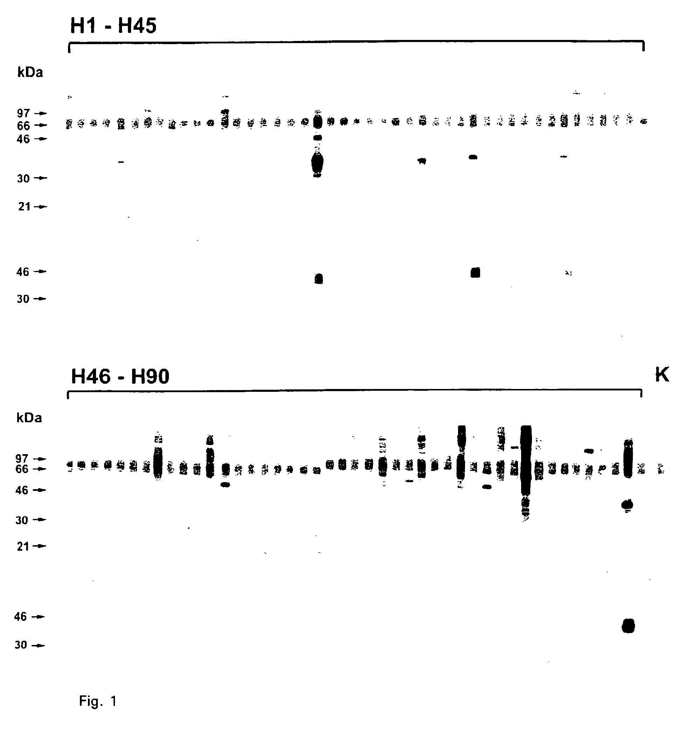

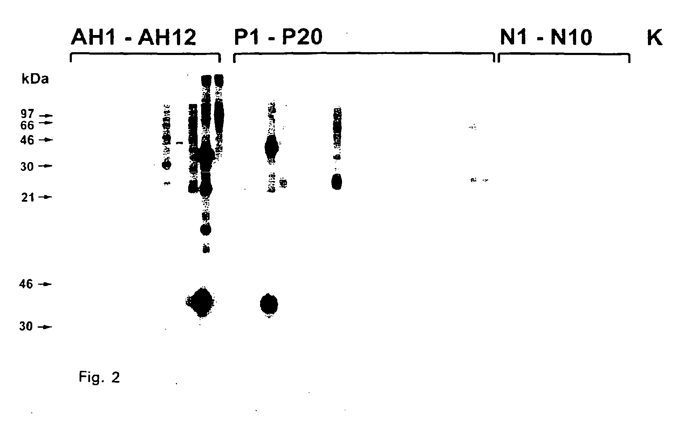

Die Figur 1 zeigt in ihrem oberen Teil die Resultate dieses Experiments für die Gruppe der "Indoor"-Allergiker (Patienten mit allergischen Beschwerden in Innenräumen), Figur. 2 zeigt im oberen Teil die Ergebnisse für die anderen drei Gruppen. Die Ergebnisse sind auch weiter unten in Tabelle 1 dargestellt.

**Tabelle 1: Zusammenfassung der IgE-Immunoblotresultate gegen verschiedene Allergenextrakte und gegen das rekombinante p40 Allergen mit Hexahistidintag aus der Dörrobstmotte. + + +, sehr starke Reaktion; + + starke Reaktion mit mindestens zwei starken positiven Banden; + schwache positive Reaktion mit mindestens einer sichtbaren Bande; - keine definierte positive Bande beobachtet.**

| **Patient** | **Dörrobst motten larven** | **Mehl motten falter** | **Rek. p40 Allergen (His₆)** | **Haus staub milbe** | **Küchen schabe** |
|---|---|---|---|---|---|
| H1 | + | + | - | | |
| H2 | - | - | - | | |
| H3 | - | - | - | | |
| H4 | - | - | - | | |
| H5 | ++ | + | - | ++ | + |
| H6 | - | - | - | | |
| H7 | ++ | ++ | - | - | - |
| H8 | - | - | - | | |
| H9 | + | + | - | - | - |
| H10 | - | - | - | | |
| H11 | - | - | - | | |
| H12 | - | - | - | | |
| H13 | ++ | ++ | - | - | ++ |
| H14 | - | - | | | |
| H15 | - | - | - | | |
| H16 | - | - | - | + | - |
| H17 | + | - | - | | |
| H18 | - | - | - | | |
| H19 | - | - | - | | |
| H20 | +++ | +++ | +++ | ++ | +++ |
| H21 | + | + | - | ++ | - |
| H22 | + | + | - | + | - |
| H23 | - | - | - | | |
| H24 | - | - | - | | |
| H25 | - | - | - | | |
| H26 | - | - | - | | |
| H27 | - | - | - | | |
| H28 | ++ | ++ | - | ++ | ++ |
| H29 | - | + | - | + | - |
| H30 | - | - | - | - | - |
| H31 | - | + | - | - | - |
| H32 | ++ | ++ | +++ | - | + |
| H33 | + | + | - | - | - |
| H34 | - | - | - | - | - |
| H35 | + | + | - | - | + |
| H36 | - | - | - | | |
| H37 | - | - | - | | |
| H38 | + | - | - | - | - |
| H39 | ++ | + | ++ | + | - |
| H40 | + | + | - | - | + |
| H41 | + | - | - | | |
| H42 | + | - | - | - | - |
| H43 | - | - | - | | |
| H44 | - | - | - | | |
| H45 | + | - | - | +++ | - |
| H46 | + | + | - | + | - |
| H47 | - | - | - | - | - |
| H48 | + | - | - | - | - |
| H49 | - | + | - | | |
| H50 | - | - | - | | |
| H51 | - | - | - | | |
| H52 | - | - | - | | |
| H53 | ++ | ++ | - | + | ++ |
| H54 | + | - | - | - | - |
| H55 | - | + | - | | |
| H56 | - | - | + | | |
| H57 | ++ | + | - | | |
| H58 | ++ | - | + | ++ | - |
| H59 | - | - | - | | |
| H60 | - | - | - | | |
| H61 | + | - | - | | |
| H62 | + | - | - | - | - |
| H63 | + | - | - | | |
| H64 | + | - | - | - | - |
| H65 | - | - | - | | |
| H66 | - | + | - | | |
| H67 | - | - | - | | |
| H68 | - | - | - | | |
| H69 | + | + | - | | |
| H70 | ++ | ++ | - | - | ++ |
| H71 | - | - | - | | |
| H72 | + | - | + | + | - |
| H73 | ++ | + | - | - | |
| H74 | - | + | - | | |
| H75 | - | - | - | | |
| H76 | +++ | ++ | - | - | ++ |
| H77 | - | - | | | |
| H78 | ++ | ++ | + | - | + |
| H79 | + | ++ | - | - | ++ |
| H80 | + | + | - | - | - |
| H81 | +++ | +++ | + | - | +++ |
| H82 | + | ++ | - | - | + |
| H83 | - | - | - | - | - |
| H84 | + | - | - | + | - |
| H85 | - | - | - | | |
| H86 | ++ | + | + | - | + |
| H87 | - | - | - | + | - |
| H88 | - | - | - | | |
| H89 | +++ | + | +++ | + | + |
| H90 | - | - | - | +++ | - |
| AH1 | - | - | - | | |
| AH2 | + | - | - | + | - |
| AH3 | + | - | - | | |
| AH4 | + | - | - | | |
| AH5 | + | + | - | - | - |
| AH6 | + | - | - | | |
| AH7 | - | - | | | |
| AH8 | ++ | ++ | + | +++ | ++ |
| AH9 | + | + | - | - | - |
| AH10 | +++ | +++ | + | +++ | ++ |
| AH11 | +++ | +++ | +++ | +++ | +++ |
| AH12 | ++ | +++ | - | +++ | + |
| P1 | + | - | - | | |
| P2 | - | - | - | | |
| P3 | - | - | - | | |
| P4 | +++ | +++ | +++ | + | +++ |
| P5 | + | + | - | ++ | + |
| P6 | - | - | - | - | - |
| P7 | + | - | - | - | + |
| P8 | - | - | - | - | |
| P9 | +++ | +++ | - | + | ++ |
| P10 | - | - | - | | |
| P11 | - | - | - | | |
| P12 | + | - | - | - | + |
| P13 | - | - | - | - | - |
| P14 | - | - | - | | |
| P15 | + | - | - | - | - |
| P16 | + | - | - | - | - |
| P17 | - | - | - | | |
| P18 | - | - | - | | |
| P19 | + | ++ | - | + | + |
| P20 | + | ++ | - | + | + |
| N1 | - | - | - | - | - |
| N2 | - | - | - | - | - |
| N3 | - | - | - | - | - |
| N4 | - | - | - | - | - |
| N5 | - | - | - | - | - |
| N6 | - | - | - | | |
| N7 | - | - | - | | |
| N8 | - | - | - | | |
| N9 | - | - | - | | |
| N10 | - | - | - | | |

Insgesamt wurden bei den "Indoor"-Allergikern (n=90) beim IgE-Immunoblot mit dem Dörrobstmottenlarven-Gesamtextrakt 4 sehr stark positive, 1 3 stark positive und 25 schwach positive Reaktionen beobachtet, bei den Atopikern mit allergischen Beschwerden in Innenräumen (n =12) 2 sehr stark positive, 2 stark positive und 6 schwach positive. Bei den Pollenallergikern ohne angegebene allergische Beschwerden in Innenräumen (n = 20) gab es 2 sehr stark positive und 8 schwach positive Reaktionen. Insgesamt wurde bei 51 % der Patienten eine positive Reaktion auf Mottenlarvenproteine beobachtet. Keine der nichtallergischen Kontrollpersonen zeigte eine Reaktion im Immunoblot.

### Test auf IgE-Antikörper gegen Proteine der Mehlmotte (E. kuehniella) im Falterstadium.

Das gleiche Patientenkollektiv wie oben wurde auf Streifchen mit einem kommerziell erhältlichen Extrakt aus der Mehlmotte untersucht, wobei die Ergebnisse auch in der Tabelle 1 dargestellt sind. Insgesamt zeigten 36 % der Allergiker eine positive Reaktion auf Mottenfalter.

### Test auf IgE-Antikörper gegen Proteine der Haustaubmilbe (Dermatophagoides pteronyssinus) und der Küchenschabe (Blattella germanica).

Ausgewählte Patienten und Normalpersonen aus dem Kollektiv wurden auf Streifchen mit kommerziell erhältlichen Extrakten aus der Hausstaubmilbe und der Küchenschabe getestet. Die Ergebnisse sind wieder in Tabelle 1 dargestellt. Die Küchenschabe ist ein flügelloses Insekt und näher mit der Dörrobstmotte verwandt als die Hausstaubmilbe, die zu den Spinnentieren zählt. Alle drei zählen zu den Gliederfüßlern (Arthropoden). Trotz der phylogenetischen Verwandtschaft der drei Spezies ist die IgE-Reaktivität der Patienten oft stark unterschiedlich. So reagiert zum Beispiel Patient H81 sehr stark auf Motte und Küchenschabe, aber nicht auf die Hausstaubmilbe. Patient H90 reagiert nur sehr stark mit der Milbe, aber nicht mit Schabe oder Motte. Patienten H7, H9, H33, H80, AH5 und AH9 reagieren auf Mottenlarven und Falter, aber nicht auf Schabe oder Milbe.

### Beispiel 2:

### Isolierung und molekulare Charakterisierung eines cDNA Klons, der für das P. interpunctella Allergen p40 kodiert

### Konstruktion einer cDNA-Bank von Plodia interpunctella.

Die Insekten (*Plodia interpunctella*) wurden in Haferflocken angezüchtet (S. Prozell, M. Schöller, Institut für Vorratsschutz, Biologische Bundesanstalt, Berlin). 180 Larven im späten Wanderstadium, kurz vor der Verpuppung (2,4 g) wurden zur Präparation von RNA eingesetzt. Die Larven wurden in 30 ml Trizol Reagens (Life Technologies, Frederick, MY, USA) homogenisiert, und aus der wäßrigen Phase wurde nach dem Protokoll des Herstellers die RNA gewonnen. Aus 5 µg der erhaltenen Gesamt-RNA wurden mit Hilfe des PolyATtract Systems (Promega, Madison, WI, USA) polyA⁺ RNA gewonnen. Die mRNA wurde in cDNA überschrieben und diese mit Hilfe des Uni-ZAP Systems (Stratagene, La Jolla, CA, USA) auf gerichtete Weise in λ ZAP Phagen eingebaut. Die primäre Bank enthielt 3 x 10⁶ cDNA Klone und wurde nach Standardmethoden amplifiziert.

### IgE-Immunoscreening und Analyse der immunopositiven Klone.

Zum Screening einer cDNA-Bank von *Plodia interpunctella* wurden Seren der Patienten AH11 (Screen 1), H20 (Screen 2) und AH10 und AH12 (Screen 3) verwendet. 360000 (Screen 1) oder 200000 (Screen 2, 3) Phagen der λ ZAP cDNA Bank wurden auf einem Rasen von *Escherichia coli* XL1-Blue (Stratagene) Zellen in einer Dichte von 15000 Phagen pro Petrischale mit 140 mm Durchmesser ausplattiert. Die Synthese von rekombinanten Proteinen wurde durch Auflegen von Nitrozellulosefiltern (Schleicher & Schüll, Dassel, Deutschland) induziert, die mit einer 10 mM IPTG (Isopropylthio-β-D-Galaktosid) Lösung getränkt waren (Huynh *et al*., 1985). 31 (Screen 1, Patient AH11) bzw. 11 (Screen 2, Patient H20) und 6 (Screen 3, Patienten AH10 und AH12) immunopositive Klone wurden jeweils mit Hilfe von Patientenseren und von ¹²⁵I-markierten, gegen humanes IgE gerichteten Antikörpern (Pharmacia & Upjohn, Uppsala, Schweden) nach etablierten Methoden (Breiteneder *et al.,* 1989; Valenta etal., 1991; Vrtala *et al.*, 1993) isoliert.

### DNA-Sequenzanalyse der immunopositiven Klone.

Aus den positiven Phagen wurden durch "in vivo excision" (Short *et al.*, 1988) mit Hilfe von Heiferphagen (Stratagene, La Jolla, CA) die entsprechenden cDNA Plasmide gewonnen und nach Standardmethoden isoliert. Die DNA wurde mit Hilfe von Thermosequenase (Amersham Pharmacia, Uppsala, Schweden) und IRD800-markierten Primern (MWG Biotech, Ebersberg) auf einem LI-COR Sequenzer (LI-COR, Lincoln, NE) analysiert. Die Basensequenzen wurden in Aminosäuresequenzen übersetzt und mit Hilfe des FastA-Programms (Pearson und Lipman, 1988) mit der SwissProt Datenbank verglichen. Alle Klone wurden mit Hilfe des GAP Programms aus dem UWGCG Programmpaket (Program Manual for the Wisconsin Package, Version 8, August 1994, Genetics Computer Group, 575 Science Drive, Madison, Wisconsin, USA 53711) miteinander verglichen. Auf diese Weise und mit Hilfe des Vergleichs zu den homologen Proteinen wurden Klone identifiziert, die einen kompletten Leserahmen aufwiesen.

Beim Screening von 360000 Phagen der λ ZAP cDNA Bank mit dem Serum AH11 wurden 31 immunopositive Klone erhalten. Die *in vivo* exzidierten Plasmide wurden zunächst mit *Eco*RI und *Xho*I gespalten und auf einem Agarosegel wurden die Schnittmuster analysiert. Alle analysierbaren Klone enthielten dieselbe cDNA. Der längste verfügbare Klon wurde sequenziert (Fig. 3) und der offene Leserahmen, der ein Polypeptid von vorhergesagten 40 kDa (p40) darstellte, wurde mit den Datenbanken verglichen. Es zeigte sich, daß das gefundene Polypeptid (Fig. 3) über die gesamte Länge mit Argininkinasen verschiedener Spezies homolog war. Argininkinasen können die terminale Phosphatgruppe von ATP auf Arginin übertragen und so einen Energiereservestoff bilden. Bislang sind Argininkinasen nicht als Allergene identifiziert worden (siehe auch Einleitung). Die dem p40 Allergen am nächsten verwandte Argininkinase aus der Honigbiene (Kucharski und Maleszka, 1998) weist 85 % Aminosäure-Sequenzidentität mit p40 auf.

### Beispiel 3:

### Isolierung und molekulare Charakterisierung eines cDNA Klons, der für das P. interpunctella Allergen p33 kodiert

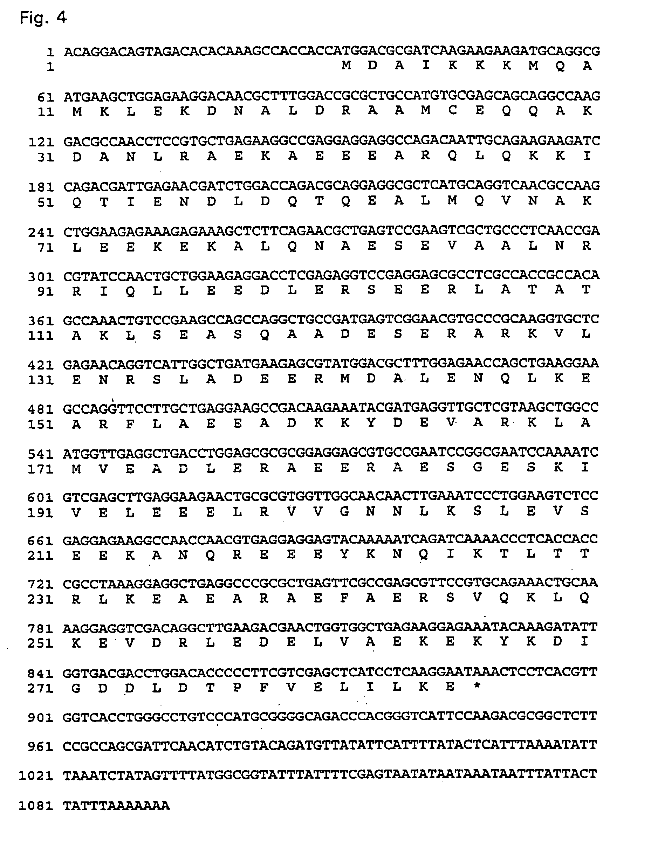

200000 Phagen der λ ZAP cDNA Bank wurden wie oben beschrieben mit dem Serum H20 gescreent, dabei wurden 11 immunopositive Klone erhalten. Die *in vivo* exzidierten Plasmide wurden wie oben analysiert und sequenziert. 10 der 11 Klone kodierten für das gleiche Protein. Drei der Klone hatten die volle Länge, und zwei von ihnen hatten die identische Sequenz, die in Fig. 4 dargestellt ist. Der offene Leserahmen stellt ein Allergen von 33 kDa dar (p33), das mit Tropomyosinen verschiedener Spezies eng verwandt ist. Tropomyosine sind als kreuzreagierende Allergene besonders auch aus dem Bereich der Nahrungsmittelallergie bekannt (Reese *et al.*, 1999).

### Beispiel 4:

### Isolierung und molekulare Charakterisierung eines cDNA Klons, der für das P. interpunctella Allergen p84 kodiert

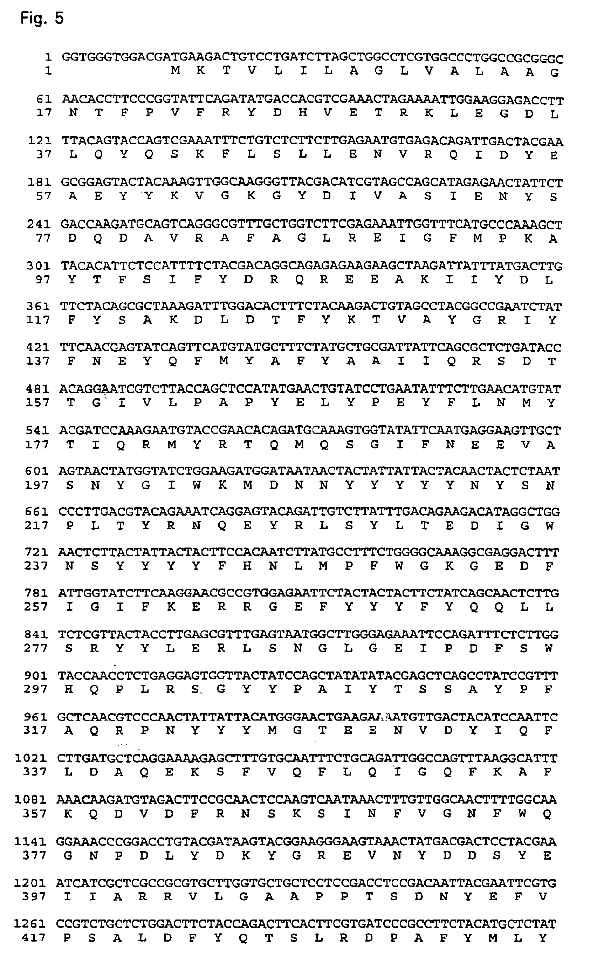

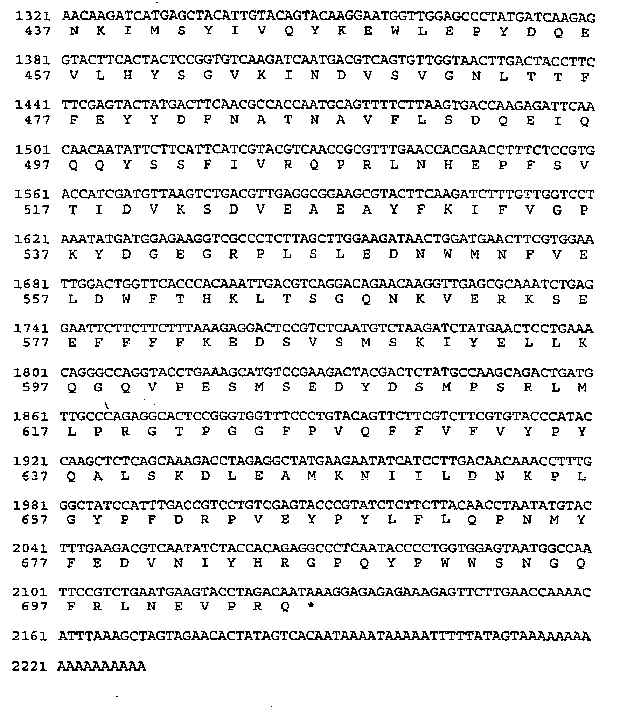

200000 Phagen der λ ZAP cDNA Bank wurden wie oben beschrieben mit den Seren AH10 und AH12 gescreent, dabei wurden 6 immunopositive Klone erhalten. Die *in vivo* exzidierten Plasmide wurden wie oben analysiert und sequenziert. Nur einer der Klone (Sequenz Fig. 5) kodierte für ein Protein in voller Länge, es handelte sich um ein homologes zu Arylphorinen. Arylphorine gelten als Speicherproteine von Insekten und enthalten einen hohen Anteil an Tyrosin. Ein Arylphorin der Schabe (*Periplaneta americana*) ist bereits in einer Publikation (Wu et al., 1996) als Allergen beschrieben.

### Beispiel 5:

### Isolierung und molekulare Charakterisierung eines cDNA Klons, der für das P. interpunctella Allergen p27 kodiert

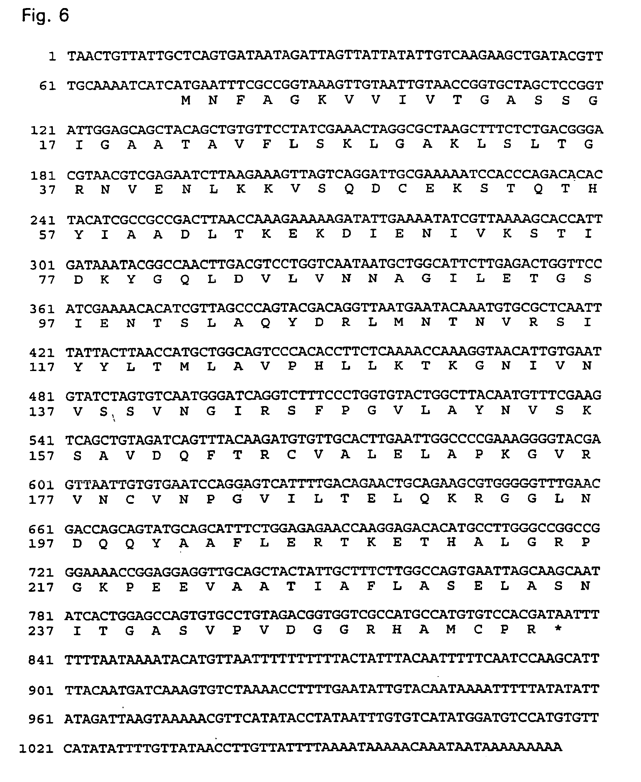

200000 Phagen der λ ZAP cDNA Bank wurden wie oben beschrieben mit dem Serum H20 gescreent (Screen 2), ebensoviele mit den Seren AH 10 und AH12 (Screen 3). Die in vivo exzidierten Plasmide wurden wie oben analysiert und sequenziert. Einer der Klone von Screen 2 und drei der Klone von Screen 3 kodierten für das gleiche Protein. Ein Sequenzvergleich der durch Übersetzung erhaltenen Aminosäuresequenz ergab eine signifikante Ähnlichkeit mit einer Glukose 1-Dehydrogenase aus *Bacillus megaterium* (36 % Sequenzidentität, Nagao *et al.*, 1992). Es gab auch eine kleinere aber noch signifikante Ähnlichkeit mit dem Alt a 2 Allergen aus dem Pilz *Alternaria alternata* (26 % Sequenzidentität, De Vouge *et al.,* 1998), einer Aldehyddehydrogenase, und dem Bet v 5 Allergen aus der Birke (20 % Sequenzidentität, Karamloo *et al.,* 1999), einer Isoflavonreduktase. Bei dem p27 Allergen handelt es sich also um ein Redoxenzym. Die Sequenz ist in Fig. 6 dargestellt.

### Beispiel 6:

### Expression des p40 Allergens mit einem Hexahistidintag und als Fusionsprotein in E. coli

Die p40 cDNA wurde auf zwei verschiedene Weisen in pET-Expressionsvektoren einkloniert so daß das p40 Allergen einmal nur mit einem Hexahistidintag und einmal als Fusionsprotein mit einer Zellulose-bindenden Domäne erzeugt wurde. Das erste Konstrukt wurde unter nativen Bedingungen gereinigt. Das zweite Konstrukt wurde über eine Zellulosesäule gereinigt. Sowohl das Fusionsprotein mit einer zellulosebindenden Domäne als auch das Nichtfusionsprotein mit Hexahistidintag besaßen Argininkinaseaktivität.

### Konstruktion eines Expressionsvektors zur Expression des p40 Allergens mit einem Hexahistidin-"Tag"

Die komplette cDNA wurde in zwei Stufen in die *Eco*RI und *Xho*I Schnittstellen des Plasmids pET23( +) (Novagen, Madison, Wisconsin, USA) einkloniert, Die Ribosomenbindungsstelle wurde mit Hilfe der Oigonukleotidabhängigen Mutagenese nach Kunkel *et al.*, (1987) in den Expressionsvektor eingebaut. Dazu wurde das Mutageneseoligonukleotid 5' - GGT AGC GGC GTC CAC CAT GGT ATA TCT CCT TCT AGA GGG AAA CCG - 3' verwendet. Der entstandene Vektor pETAK1 wurde durch DNA-Sequenzanalyse überprüft. In dem Vektor wurde dann am carboxyterminalen Ende der Sequenz durch eine zweite Mutagenese mit dem Oligonukleotid 5'-ATC TCA GTG GTG GTG GTG GTG GTG CAG GGA TTT CTC GAT TTT GAT-3' ein Hexahistidintag für die Reinigung über eine Nickelaffinitätssäule (Qiagen, Hilden, Deutschland) in das Expressionsplasmid eingebracht, und es entstand der Vektor pETHisAK1, der durch Sequenzierung überprüft wurde.

### Expression und Reinigung des p40 Allergens als Nichtfusionsprotein mit einem Hexahistidin-"Tag"

Der Vektor pETHisAK1 wurde in *Escherichia coli* BL21 (DE3) transformiert und die transformierten Zellen in einer 400 ml Kultur bei 37 °C geschüttelt bis zu einer optischen Dichte (600 nm) von 0,8. Die Synthese des rekombinanten Proteins wurde durch Zugabe von 0,4 mM (Endkonzentration) von IPTG (Isopropyl-β-D-Galaktosid) induziert, und die Kultur wurde noch 3 h bei 37 °C weitergeschüttelt. Die Zellen wurden durch Zentrifugation geerntet, dann wurden die Zellen 10 min in Lysepuffer (100 mM KCI, 50 mM Mes, 4 % (v/v) Triton X-100, 8 mM DTT, 8 mM EDTA, 25 µg/ml Polymyxin B Suifat (Sigma, St. Louis, MO, USA), pH 7.5) behandelt (Schupp *et al.*, 1995). Zelldetritus wurde 30 min bei 2000 x g und 4 °C abzentrifugiert. Das Protein wurde mit Hilfe von zentrifugierbaren Kleinsäulen unter nativen Bedingungen durch Nickelchelat-Affinitätschromatographie gereinigt wie vom Hersteller (Qiagen) beschrieben.

### Konstruktion eines Expressionsklons zur Expression des p40 Allergens als Fusionsprotein

Die komplette cDNA, die für das p40 Allergen kodiert, wurde in den Expressionsvektor pET36b (Novagen) nach Standardmethoden (Sambrook *et al.*, 1989) unter Verwendung der *Eco*RI und *Xho*I Restriktionsstellen umkloniert. Dies geschah in zwei Stufen, da die cDNA eine interne *Xho*I-Stelle aufwies. Der noch fehlende Übergang zwischen der Sequenz, die für die Zellulose bindende Domäne kodierte und der Sequenz, die für die Argininkinase kodierte, wurde mit Hilfe der Oligonukleotid-abhängigen Mutagenese nach Kunkel *et al.,* (1987) in den Expressionsvektor eingebaut.

Dazu wurde das Mutageneseoligonukleotid 5 '- GGT AGC GGC GTC CAC CAT GGT ATA TCT CCT TCT AGA GGG AAA CCG - 3' verwendet. Der entstandene Vektor pCBDAK1 wurde durch DNA-Sequenzanalyse überprüft.

### Expression eines Fusionsproteins aus einer Zellulose-bindenden Domäne und dem p40 Allergen

Der Vektor pCBDAK1 wurde in *Escherichia coli* BL21 (DE3) transformiert und die transformierten Zellen in einer 400 ml Kultur bei 37°C geschüttelt bis zu einer optischen Dichte (600 nm) von 0,8. Die Synthese des rekombinanten Proteins wurde durch Zugabe von 0,4 mM (Endkonzentration) von IPTG (Isopropyl-β-D-Galaktosid) induziert, und die Kultur wurde noch 3 h bei 37°C weitergeschüttelt. Die Zellen wurden durch Zentrifugation geerntet, dann wurden die Zellen 10 min in Lysepuffer (100 mM KCi, 50 mM Mes, 4 % (v/v) Triton X-100, 8 mM DTT, 8 mM EDTA, 25 µg/ml Polymyxin B Sulfat (Sigma, St. Louis, MO, USA), pH 7.5) behandelt (Schupp *et al*., 1995). Zelldetritus wurde 30 min bei 2000 x g und 4 °C abzentrifugiert. Das Fusionsprotein wurde durch Zellulose-Affinitätschromatographie gereinigt wie vom Hersteller (Novagen) beschrieben.

### Test des rekombinanten p40 Allergens mit Hexahistidintag und des Fusionsproteins aus einer Zellulose-bindenden Domäne und dem p40 Allergen auf IgE-Reaktivität.

Das gereinigte p40 Allergen mit einem Hexahistidintag wurde wie oben beschrieben in einer Konzentration von 10 µg pro cm auf einem präparativen 12,5 % SDS-Polyacrylamidgel elektrophoretisiert, auf Nitrozellulose geblottet und mit jenen Patientenseren getestet, die in den oben beschriebenen Experimenten mit dem Extrakt von *P. interpunctella* Larven getestet worden waren.

Das gereinigte rekombinante Fusionsprotein wurde wie oben beschrieben in einer Konzentration von 5 µg pro cm auf einem präparativen 12,5 % SDS-Polyacrylamidgel elektrophoretisiert, auf Nitrozellulose geblottet und mit jenen Patientenseren getestet, die in den oben beschriebenen Experimenten mit dem Extrakt von *P. interpunctella* Larven positive Signale ergeben hatten.

### Test der oben beschriebenen Patienten und Normalpersonen auf IgE-Antikörper gegen das rekombinante p40 Allergen mit Hexahistidintag.

Alle Seren der oben beschriebenen Patienten und Normalpersonen wurden auf gleiche Weise wie oben beschrieben auf IgE-Antikörper gegen das gereinigte p40 Allergen mit Hexahistidintag getestet (Fig. 1, 2, Tabelle 1). Bei diesem Versuch zeigte sich eine Reaktivität nur im Molekulargewichtsbereich bei 40 kDa, deshalb ist ein schmalerer Ausschnitt der Immunoblots unter den Blots mit Larvenproteinen dargestellt. Insgesamt waren 10 von 90 "Indoor"-Allergikern positiv, 3 von 12 Atopikern mit "Indoor"-Allergie und einer von 20 Pollenallergikern ohne angegebene allergischen Beschwerden in Innenräumen. Das bedeutet, dass 11 % der Patienten H1-H90 und 23 % der Patienten AH 1-AH 12 IgE gegen Larven der Dörrobstmotte hatten.

### Test von Patienten und Normalpersonen auf IgE-Antikörper gegen das rekombinante p40 Fusionsprotein mit Zellulose-bindender Domäne.

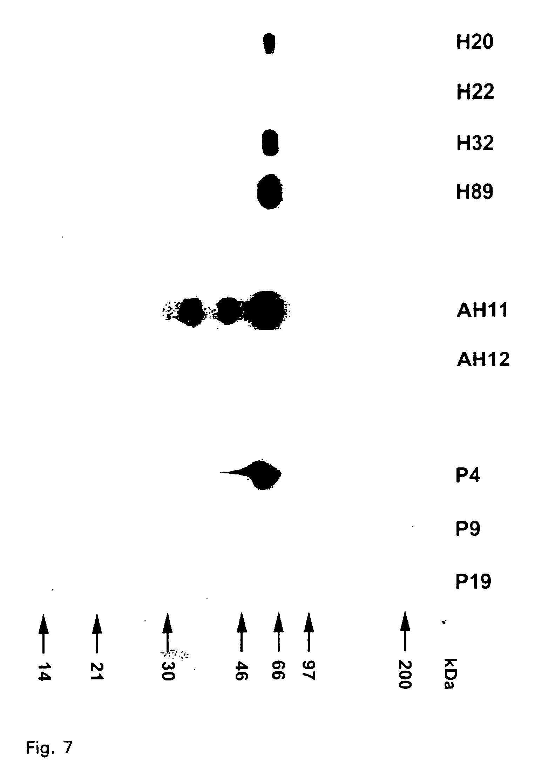

Eine Auswahl der oben beschriebenen Seren wurde auf IgE gegen das rekombinante p40 Fusionsprotein getestet (Beispiele in Fig. 7). Auch das rekombinante p40 Fusionsprotein war geeignet, IgE-Antikörper gegen das natürliche p40 Antigen nachzuweisen.

### Beispiel 7:

### Hauttests

Das gereinigte rekombinante p40 Allergen mit Hexahistidintag wurde in steriler0,9 % NaCI-Lösung auf 10 verschiedene Konzentrationen eingestellt: Nr. 1 enthielt 400 ng/µl, die weiteren Proben enthielten absteigende Konzentrationen von 200 ng/µl (Nr. 2), 100 ng/µl (Nr. 3), 50 ng/µl (Nr. 4), 25 ng/ul (Nr. 5), 12,5 ng/ul (Nr. 6), 6,25 ng/µl (Nr. 7), 3,13 ng/µl (Nr. 8), 1,56 ng/µl (Nr. 9) und 0,78 ng/µl (Nr. 10). Vor dem intrakutanen Hauttest wurde am kontralateralen Arm ein Reibetest mit je 30 µl der Konzentrationen Nr. 10 bis Nr. 2 durchgeführt und nach 5, 10 und 20 min sowie 24 h abgelesen. Die Negativkontrolle war 0,9 % NaCI, als Positivkontrolle wurde Histamindihydrochlorid in einer Konzentration von 1 mg/mi verwendet. Basierend auf den Ergebnissen des Reibetests wurde dann der Pricktest in den Konzentrationen Nr. 10 bis Nr. 5 durchgeführt und jeweils nach 20 min und 24 h abgelesen.

Der mottenallergische Patient AH 11 wurde gegen das rekombinante p40 Allergen mit Hexahistidintag zuerst im Reibetest und dann im Pricktest untersucht. Im Reibetest (Fig. 8b) riefen die Konzentrationen Nr. 10 bis Nr. 6 keine Sofortreaktionen hervor, die Konzentration Nr. 5 induzierte einen leichten Juckreiz im Probegebiet. Nr. 4 rief nach 5-10 Minuten winzige Quaddeln hervor. Nr. 3 und Nr. 2 riefen multiple Quaddeln (Durchmesser 4-5 mm) hervor. Diese hatten nach 15-20 min die maximale Ausprägung (Fig. 8d) und bildeten sich alle nach 45 min zurück.

Nach dem Reibetest wurde der Pricktest mit den gleichen Verdünnungen (Nr. 10 bis Nr. 5) in aufsteigenden Konzentrationen durchgeführt (Fig. 8a). Auf die Konzentrationen Nr. 10 und Nr. 9 wurde keine unmittelbare Hautreaktion beobachtet. Qaddeln und Hautrötung traten in den Konzentrationen Nr. 8 (3,12 ng/µl) bis Nr. 5 (25 ng/µl) auf. Der Durchmesser der Quaddeln war zwischen 7 mm (Konzentration Nr. 8) und 15 mm (Konzentration Nr. 6) (Fig. 8c). Aufgrund der Stärke der Reaktionen von den Konzentrationen Nr. 8 bis Nr. 5 wurden Konzentrationen Nr. 4 bis Nr. 1 nicht getestet. Die Quaddeln wurden zur Dokumentation bei ihrer maximalen Ausprägung nach 20 min mit einem Stift markiert und bildeten sich nach 45 min spontan zurück.

Bei der Ablesung nach 24 h wurden innerhalb der markierten Grenzen der vorangegangenen Soforttyp-Reaktionen akzematoide Papeln als Spätphasenreaktion des Pricktests beobachtet (Fig. 9d). Der kontralaterale Arm, an dem der Reibetest durchgeführt worden war, zeigte eine ausgeprägte ekzematöse Reaktion im Gebiet der Konzentrationen Nr. 6 bis Nr. 4 (Fig. 9 a, c), während bei den niedrigeren Konzentrationen Nr. 10 bis Nr. 7 keine ekzematöse Reaktion beobachtet wurde (Fig. 9b).

### Beispiel 8:

### Immunoblot-Inhibition und Nachweis der Kreuzreaktivität des p40 Allergens mit Allergenen verschiedener Spezies

Aus der Literatur ist bekannt, daß eine enzymatische Argininkinaseaktivität praktisch in allen untersuchten Invertebraten vorkommt. Um zu überprüfen, welche immunologische Ähnlichkeiten zwischen dem p40 Allergen der Dörrobstmotte und den Homologen in anderen Spezies bestehen, wurde ein Immunoblot-Inhibitionsexperiment durchgeführt. Allergenextrakte aus der Milbe (*Dermatophagoides pteronyssinus*), Küchenschabe (*Blattella germanica*), Garnele (*Penaeus monodon),* Hummer (*Homarus gammarus),* Miesmuschel (*Mytilus edulis*), und Kabeljau (*Gadus morhua*) als einzigem Vertebraten wurden entweder eingekauft (Milbe und Schabe) oder aus frisch eingekauftem, ungekochten Muskelfleisch präpariert.

Die Gesamtallergene von der Milbe und der Küchenschabe stammten von Pharmacia/Allergon. Die verschiedenen Meeresfrüchte wurden in frischem, ungekochten Zustand auf dem Naschmarkt in Wien erworben. Es wurde so gut wie möglich nur Muskelfleisch verarbeitet. Die verschiedenen Proben (1 - 5 g) wurden in flüssigem Stickstoff eingefroren, in der Reibschale zerrieben, mit 3 ml pro g Probe in eiskaltem bidest. H₂O mit 5 mM PMSF (Phenylmethansulfonylfluorid) 1 h bei 4 °C gerührt. Ein Volumen Auftragspuffer (Fling und Gregerson, 1986) wurde zugesetzt und die Proben wurden 10 min bei 95 °C denaturiert, unlösliche Bestandteile wurden 10 min bei 14500 Upm und 4 °C abzentrifugiert, und die Proteinkonzentration der Extrakte wurde auf einem Coomassie-gefärbten SDS-Polyacrylamidgel abgeschätzt. Wie oben wurden präparative Gele mit 200µg Protein pro cm gefahren, auf Nitrozellulose geblottet und in Streifen geschnitten. Das Serum des Patienten AH11 und von den Patienten H89 und H32 wurde in der Konzentration 1 :10 mit Puffer G (42 mM Na₂HPO₄, 6,4 mM NaH₂PO₄, 0,5 % (v/v) Tween 20, 0,5 % (w/v) Rinderserumalbumin, 0,05 % (w/v) NaN₃, pH 7.5) verdünnt. Je 1 ml der Proben wurde entweder mit 10 µg des rekombinanten p40 Allergens mit Hexahistidintag in Puffer G, oder nur in Puffer G über Nacht bei 4 °C präinkubiert, und dann wurde je mit einem Streifen Nitrozellulose mit geblotteten Extrakten die IgE-Reaktivität bestimmt. Das gebundene IgE wurde wie üblich mit jodmarkierten Antihuman-IgE Antikörpern detektiert.

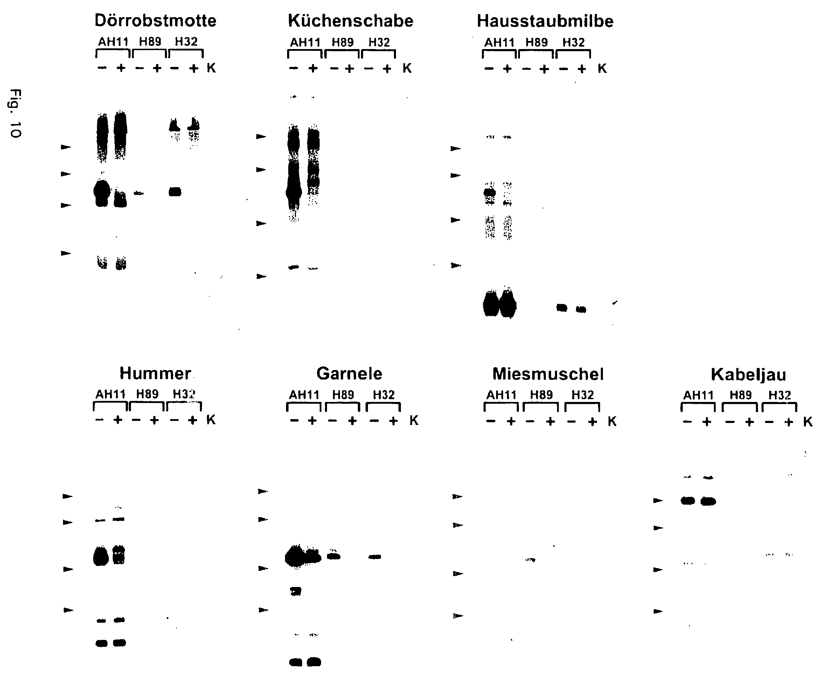

Bei allen untersuchten Invertebratenspezies reagierten die Seren mit einer Bande im Bereich von 40 kDa, die durch Präinkubation mit dem rekombinanten p40 Allergen aus der Dörrobstmotte entweder ausgelöscht (Dörrobstmotte, Hausstaubmilbe) oder abgeschwächt (Küchenschabe, Garnele, Hummer, Miesmuschel) wurde (Fig. 10). Im Extrakt aus Kabeljau gab es zwar eine Reihe von allergenen Proteinen, aber keines von ihnen wurde durch Präinkubation mit dem p40 Allergen aus der Motte teilweise oder vollständig inhibiert.

### LITERATUR

Anisike E O, Moreland B H, Watts D C (1975) Evolutionary variation between a monomer and a dimer arginine kinase. Biochem. J. 145: 535-543.
Arruda L K, Vailes L D, Benjamin D C, Chapman M D (1995) Molecular cloning of German cockroach (Blattella germanica) allergens. Int. Arch. Allergy Immunol. 107: 295-297.
Baldo B A, Panzani R C (1988) Detection of IgE antibodies to a wide range of insect species in subjects with suspected inhalant allergies to insects. Int. Arch. Allergy Appl. Immunol. 85: 278-287.
Breiteneder H, Pettenburger K, Bito A, Valenta R, Kraft D, Rumpold H, Scheiner O, Breitenbach M (1989) The gene coding for the major birch pollen allergen Bet v 1, is highly homologous to a pea disease resistance response gene. EMBO J. 8: 1935-1938.
Davis F M, Jenkins J N (1995) Management of scales and other insect debris: occupational health hazard in a lepidopterous rearing facility. J. Econ. Entomol. 88: 185-191.
De Vouge M W, Thaker A J; Zhang L, Muradia G, Rode H, Vijay H M (1998) Molecular cloning of IgE-binding fragments of Alternaria alternata allergens. Int. Arch. Allergy Immunol. 116: 261-268.
Fling S P, Gregerson D S (1986) Peptide and protein molecular weight determination by electrophoresis using a high-molarity tris buffer system without urea. Anal. Biochem. 155: 83-8.
Huynh T V et al., In: cDNA cloning, Oxford, IRL Press, 1 (1985) 49-78.
Jarolim E, Rumpold H, Endler A T, Ebner H, Breitenbach M, Scheiner O, Kraft D (1989) IgE and IgG antibodies of patients with allergy to birch pollen as tools to define the allergen profile of Betula verrucosa. Allergy 44: 385-395.
Karamloo F, Schmitz N, Scheurer S, Foetisch K, Hoffmann A, Haustein D, Vieths S (1999) Molecular cloning and characterization of a birch pollen minor allergen, Bet v 5, belonging to a family of isoflavone reductaserelated proteins. J. Allergy Clin. Immunol. 104: 991-999.
Komase Y, Sakata M, Azuma T, Tanaka A, Nakagawa T (1997) IgE antibodies against midge and moth found in Japanese asthmatic subjects and comparison of allergenicity between these insects. Allergy 52 : 75-81.
Kraft D, Ferreira F, Vrtala S, Breiteneder H, Ebner C, Valenta R, Susani M, Breitenbach M, Scheiner O (1999) The importance of recombinant allergens for diagnosis and therapy of IgE-mediated allergies. Int. Arch. Allergy Immunol. 118: 171-176.
Kucharski R, Maleszka R (1998) Arginine kinase is highly expressed in the compound eye of the honey bee, Apis mellifera. Gene 211: 343-349.
Kunkel T A, Roberts J D, Zakour R A (1987) Rapid and efficient site-specific mutagenesis without phenotypic selection. Methods Enzymol. 154: 367-382.
Lin R-Y, Shen H-D, Han S-H (1993) Identification and characterization of a 30 kd major allergen from Parapenaeus fissurus. J. Allergy Clin. Immunol. 92: 837-845.
Miyamoto T, In: Advances in Allergology and Clinical Immunology, Eds Godard P et al., The Parthenon Publishing Group-Carnforth, U.K. and New Jersey, USA, (1992) 343-347.
Nagao T, Mitamura T, Wang X H, Negoro S, Yomo T, Urabe I, Okada H (1992) Cloning, nucleotide sequences, and enzymatic properties of glucose dehydrogenase isozymes from Bacillus megaterium IAM1030. J. Bacteriol. 174: 5013-5020.
Pearson W R, Lipman D J (1988) Improved tools for biological sequence comparison. Proc. Natl. Acad. Sci. USA 85 : 2444-2448.
Reese G, Ayuso R, Lehrer S B (1999) Tropomyosin: an invertebrate panallergen. Int. Arch. Allergy Immunol. 119: 247-258.
Rosenstreich D L, Eggleston P, Kattan M, Baker D, Slavin R G, Gergen P, Mitchell H, McNiff Mortimer K, Lynn H, Ownby D, Malveaux F (1997) The role of cockroach allergy and exposure to cockroach allergen in causing morbidity among inner-city children with asthma. N. Engl. J. Med. 336: 1356-1363.
Schupp J M, Travis SE, Price L B, Shand R F, Keim P (1995) Rapid bacterial permeabilization reagent useful for enzyme assays. Biotechniques 19 : 18-20.
Segal D M, Taurog J D, Metzger H (1977) Dimeric immunoglobulin E serves as a unit signal for mast cell degranulation. Proc. Natl. Acad. Sci. USA 74: 2993-2997.
Short J M, Fernandez J M, Sorge J A, Huse W D (1988) Lambda ZAP: a bacteriophage lambda expression vector with in vivo excision properties. Nucleic Acids Res. 16: 7583-7600.
Storms W W, Berry C, Withee W (1981) Miller moth asthma. Clin. Allergy 11: 55-59.
Suzuki M, Itoh H, Sugiyama K, Takagi I, Nishimura J, Kato K, Mamiya S, Baba S, Ohya Y et al. (1995) Causative allergens of allergic rhinitis in Japan with special reference to silkworm moth allergen. Allergy 50: 23-27.
Thomas W R, Smith W (1999) Towards defining the full spectrum of important house dust mite allergens. Clin. Exp. Allergy 29: 1583-1587.
Towbin H, Staehelin T, Gordon J (1979) Electrophoretic transfer of proteins from polyacrylamide gels to nitrocellulose sheets: procedure and some applications. Proc. Natl. Acad. Sci. USA 76: 4350-4354.
Unger A, Stoger P, Simon Nobbe B, Susani M, Crameri R, Ebner C, Hintner H, Breitenbach M (1999) Clinical testing of recombinant allergens of the mold Alternaria alternata. Int. Arch. Allergy Immunol. 118: 220-221.
Valenta R, Duchene M, Pettenburger K, Sillaber C, Valent P, Bettelheim P, Breitenbach M, Rumpold H, Kraft D, Scheiner O (1991) Identification of profilin as a novel pollen allergen; IgE autoreactivity in sensitized individuals. Science 253: 557-560.
Valenta R, Lidholm J, Niederberger V, Hayek B, Kraft D, Gronlund H (1999) The recombinant allergen-based concept of component-resolved diagnostics and immunotherapy (CRD and CRIT). Clin. Exp. Allergy 29 (1999) 896-904.
Van Wijnen J H, Verhoeff A P, Mulder Folkerts D K, Brachel H J, Schou C (1997) Cockroach allergen in house dust. Allergy 52: 460-464.
Vrtala S, Sperr W R, Reimitzer I, van Ree R, Laffer S, Muller W D, Valent P, Lechner K, Rumpold H, Kraft D, et al. (1993) cDNA cloning of a major allergen from timothy grass (Phleum pratense) pollen; characterization of the recombinant Phl pV allergen. J. Immunol. 151: 4773-4781
Wang X, Zheng S, Zhang H (1994) A study of occupational asthma and specific IgE in sericulture workers. Chung Kuo I Hsueh Ko Hsueh Yuan Hsueh Pao. 16: 323-327
Wu C H, Lee M F, Liao S C, Luo S F (1996) Sequencing analysis of cDNA clones encoding the American cockroach Cr-PI allergens. Homology with insect hemolymph proteins. J. Biol. Chem. 271: 17937-17943.
Wyss M, Maughan D, Wallimann T (1995) Re-evaluation of the structure and physiological function of guanidino kinases in fruitfly (Drosophila), sea urchin (Psammechinus miliaris) and man. Biochem. J. 309: 255-261.

## Patentansprüche

1. Nukleinsäure, kodierend für ein allergenes Polypeptid aus Anthropoden, umfassend
(a) die in SEQ ID NO: 1 dargestellte Sequenz,
(b) eine von einer Sequenz gemäß (a) auf Grund der Degeneration des genetischen Codes abweichende Sequenz, oder/und
(c) eine Sequenz mit einer Identität > 80 % zu einer der Sequenzen von (a) oder/und (b).

2. Nukleinsäure, umfassend einen Bereich, der für ein Polypeptid mit der in SEQ ID NO: 2 dargestellten Sequenz kodiert.

3. Vektor, umfassend eine Nukleinsäure nach Anspruch 1 oder 2 in operativer Verknüpfung mit einer Expressionskontrollsequenz.

4. Rekombinanter Expressionsvektor, der eine Expressionskontrollsequenz besitzt, die funktionell mit einer Nukleotidsequenz gemäß Anspruch 1 verknüpft ist.

5. Zelle, transformiert mit einem Vektor nach Anspruch 3 oder 4.

6. Allergenes Polypeptid, kodiert durch eine der Nukleinsäuren nach Anspruch 1 oder 2.

7. Allergenes Polypeptid nach Anspruch 6 mit der in SEQ ID NO: 2 dargestellten Aminosäuresequenz.

8. Allergenes Polypeptid nach Anspruch 6 oder 7, **dadurch gekennzeichnet, dass** es mit einem heterologen Peptid oder Polypeptid fusioniert ist.

9. Allergenes Polypeptid nach Anspruch 8, **dadurch gekennzeichnet, dass** das heterologe Peptid oder Polypeptid eine zellulosebindende Domäne, ß-Galaktosidase oder Glutathion S-Transferase ist.

10. Verwendung eines Polypeptids, kodiert durch eine Nukleinsäure, welche
(a) die in SEQ ID NO: 1 dargestellte Sequenz,
(b) eine von einer Sequenz gemäß (a) auf Grund der Degeneration des genetischen Codes abweichende Sequenz, oder
(c) eine Sequenz mit einer Identität > 80% zu einer der Sequenzen von (a) oder/und (b),
umfasst, als Immunogen zur Herstellung von Antikörpern.

11. Antikörper gegen ein allergenes Polypeptid nach einem der Ansprüche 6-9, wobei der Antikörper für eine allergene Determinante des allergenen Polypeptids spezifisch ist.

12. Zusammensetzung zur Verwendung als Arzneimittel, umfassend:
(a) eine Nukleinsäure, kodierend für ein allergenes Polypeptid, umfassend
(i) die in SEQ ID NO: 1 dargestellte Sequenz,
(ii) eine von einer Sequenz gemäß (i) auf Grund der Degeneration des genetischen Codes abweichende Sequenz,
(iii) eine Sequenz mit einer Identität > 80% zu einer der Sequenzen von (i) oder/und (ii), oder
(iv) eine Sequenz, die mit einer der Sequenzen gemäß (i), (ii) oder/und (iii) unter stringenten Bedingungen hybridisiert,
(b) einen rekombinanten Vektor, umfassend eine Nukleinsäure gemäß (a) in operativer Verknüpfung mit einer Expressionskontrollsequenz,
(c) eine Zelle, transformiert mit einem Vektor gemäß (b),
(d) ein Polypeptid, kodiert durch eine Nukleinsäure gemäß (a), oder/und
(e) einen Antikörper gegen ein Polypeptid gemäß (d).

13. Verwendung einer Zusammensetzung nach Anspruch 12 zur Herstellung eines diagnostischen und/oder therapeutischen Mittels.

14. Verwendung nach Anspruch 13 für die Therapie oder/und Diagnose von allergischen Erkrankungen.

15. In vitro-Verfahren zur Diagnose einer Allergie gegen Arthropodenproteine, wobei man eine Probe einer Körperflüssigkeit aus dem Patienten, in der Antikörper gegen das Arthropodenprotein vermutet werden, mit einem Polypeptid, kodiert durch eine Nukleinsäure, welche
(a) die in SEQ ID NO: 1 dargestellte Sequenz,
(b) eine von einer Sequenz gemäß (a) auf Grund der Degeneration des genetischen Codes abweichende Sequenz,
(c) eine Sequenz mit einer Identität > 80% zu einer der Sequenzen von (a) oder/und (b), oder
(d) eine Sequenz, die mit einer der Sequenzen gemäß (a), (b) oder/und (c) unter stringenten Bedingungen hybridisiert, umfasst,
unter Bedingungen in Kontakt bringt, die die Bildung eines Komplexes zwischen dem Antikörper und dem Polypeptid ermöglichen, wonach der Komplex gemessen wird und zu der Menge des Antikörpers in der Probe in Beziehung gesetzt wird, wobei ein erhöhter Spiegel als Zeichen einer Allergie gegen das Arthropodenprotein gewertet wird, die das Polypeptid enthält.

16. In vitro-Verfahren zur Messung einer zellulären Immunreaktion, wobei ein rekombinantes oder synthetisches Protein oder Polypeptidkodiert durch eine Nukleinsäure, welche
(a) die in SEQ ID NO: 1 dargestellte Sequenz,
(b) eine von einer Sequenz gemäß (a) auf Grund der Degeneration des genetischen Codes abweichende Sequenz,
(c) eine Sequenz mit einer Identität 80% zu einer der Sequenzen von (a) oder/und (b), oder
(d) eine Sequenz, die mit einer der Sequenzen gemäß (a), (b) oder/und (c) unter stringenten Bedingungen hybridisiert, umfasst,
zur Stimulation der zellulären Immunreaktion verwendet wird.

17. Verfahren zur Bestimmung von Arthropodenallergenen in Proben aus der Umwelt des Menschen, **dadurch gekennzeichnet, dass** man die Argininkinaseaktivität (EC 2.7.3.3) in den Proben bestimmt.

18. Verfahren zur Bestimmung von Arthropodenallergenen in Proben aus der Umwelt des Menschen, **dadurch gekennzeichnet, dass** man das Vorhandensein
(a) einer Nukleinsäurekodierend für ein allergenes Polypeptid, umfassend
(i) die in SEQ ID NO: 1 dargestellte Sequenz,
(ii) eine von einer Sequenz gemäß (i) auf Grund der Degeneration des genetischen Codes abweichende Sequenz,
(iii) eine Sequenz mit einer Identität > 80% zu einer der Sequenzen von (i) oder/und (ii), oder
(iv) eine Sequenz, die mit einer der Sequenzen gemäß (i), (ii) oder/und (iii) unter stringenten Bedingungen hybridisiert, oder
(b) eines allergenen Polypeptids, kodiert durch eine Nukleinsäure gemäß (a),
bestimmt.

19. Verfahren zur Bestimmung von Arthropodenallergenen in Proben aus der Umwelt des Menschen, **dadurch gekennzeichnet, dass** man das Vorhandensein von Argininkinase aus Plodia interpunctella gemäß SEQ ID NO: 2 oder einer Argininkinase mit einer Sequenzidentität > 70% hierzu bestimmt.

20. Verfahren nach Anspruch 19, **dadurch gekennzeichnet, dass** man das Vorhandensein einer Argininkinase aus Milbe oder Motte bestimmt.

21. Verfahren zur Herstellung eines Arzneimittels, das ein synthetisches oder rekombinantes Polypeptid kodiert durch eine Nukleinsäure, welche
(a) die in SEQ ID NO: 1 dargestellte Sequenz,
(b) eine von einer Sequenz gemäß (a) auf Grund der Degeneration des genetischen Codes abweichende Sequenz,
(c) eine Sequenz mit einer Identität > 80% zu einer der Sequenzen von (a) oder/und (b), oder
(d) eine Sequenz, die mit einer der Sequenzen gemäß (a), (b) oder/und (c) unter stringenten Bedingungen hybridisiert, umfasst,
enthält und zur Hyposensibilislerung (Immuntherapie) von Patienten mit Allergie gegen Argininkinase aus Plodia interpunctella gemäß SEQ ID NO: 2 oder einer Argininkinase mit einer Sequenzidentität > 70% hierzu eingesetzt werden kann.

22. Verwendung von Argininkinase aus Plodia interpunctella gemäß SEQ ID NO: 2 oder einer Argininkinase mit einer Sequenzidentität > 70% hierzu zur Herstellung eines Arzneimittels oder/und eines diagnostischen Mittels zur Behandlung von allergischen Erkrankungen oder/und zur Bestimmung von Allergenen.

23. Verwendung nach Anspruch 22, **dadurch gekennzeichnet, dass** eine Argininkinase aus einer Motte oder aus einer Milbe verwendet oder bestimmt wird.

24. In vitro-Verfahren zum Nachweis einer Allergie, bei dem Argininkinase aus Plodia interpunctella gemäß SEQ ID NO: 2 zur Bestimmung der Allergie eingesetzt wird.

25. Allergen zur Verwendung als Arzneimittel, **dadurch gekennzeichnet, dass** es sich bei dem Allergen um Argininkinase aus Plodia interpunctella gemäß SEQ ID NO: 2 oder eine Argininkinase mit einer Sequenzidentität > 70% hierzu handelt.

## Claims

1. A nucleic acid encoding an allergenic polypeptide from arthropods, comprising
(a) the sequence represented in SEQ ID NO: 1,
(b) a sequence deviating from a sequence according to (a) on the basis of degeneration of the genetic code, and/or
(c) a sequence with an identity > 80 % to one of the sequences of (a) and/or (b).

2. A nucleic acid comprising a region which encodes a polypeptide with the sequence represented in SEQ ID NO: 2.

3. A vector comprising a nucleic acid according to Claim 1 or 2 in operative combination with an expression control sequence.

4. A recombinant expression vector which has an expression control sequence which is combined functionally with a nucleotide sequence according to Claim 1.

5. A cell transformed with a vector according to Claim 3 or 4.

6. An allergenic polypeptide encoded by one of the nucleic acids according to Claim 1 or 2.

7. An allergenic polypeptide according to Claim 6, with the amino acid sequence represented in SEQ ID NO: 2.

8. An allergenic polypeptide according to Claim 6 or Claim 7, **characterised in that** it is fused with a heterologous peptide or polypeptide.

9. An allergenic polypeptide according to Claim 8, **characterised in that** the heterologous peptide or polypeptide is a cellulose-binding domain, -galactosidase or glutathion S-transferase.

10. Use of a polypeptide encoded by a nucleic acid which comprises
(a) the sequence represented in SEQ ID NO: 1,
(b) a sequence deviating from a sequence according to (a) on the basis of degeneration of the genetic code, or
(c) a sequence with an identity > 80 % to one of the sequences of (a) and/or (b),
as immunogen for the preparation of antibodies.

11. An antibody against an allergenic polypeptide according to any one of Claims 6 to 9, wherein the antibody is specific for an allergenic determinant of the allergenic polypeptide.

12. A composition for use as a medicament, comprising:
(a) a nucleic acid encoding an allergenic polypeptide, comprising
(i) the sequence represented in SEQ ID NO: 1,
(ii) a sequence deviating from a sequence according to (i) on the basis of degeneration of the genetic code,
(iii) a sequence with an identity > 80 % to one of the sequences of (i) and/or (ii), or
(iv) a sequence which hybridises with one of the sequences according to (i), (ii) and/or (iii) under stringent conditions,
(b) a recombinant vector, comprising nucleic acid according to (a) in operative combination with an expression control sequence,
(c) a cell transformed with a vector according to (b),
(d) a polypeptide encoded by a nucleic acid according to (a), and/or
(e) an antibody against a polypeptide according to (d).

13. Use of a composition according to Claim 12 for the preparation of a diagnostic and/or therapeutic agent.

14. Use according to Claim 13 for the therapy and/or diagnosis of allergenic diseases.

15. An in-vetro method for the diagnosis of an allergy to arthropod proteins, wherein a sample of a body fluid from the patient, in which it is suspected there are antibodies to the arthropod protein, with a polypeptide, encoded by a nucleic acid which comprises
(a) the sequence represented in SEQ ID NO: 1,
(b) a sequence deviating from a sequence according to (a) on the basis of degeneration of the genetic code,
(c) a sequence with an identity > 80 % to one of the sequences of (a) and/or (b), or
(d) a sequence which hybridises with one of the sequences according to (a),(b) and/or (c) under stringent conditions,
is brought into contact under conditions which make possible the formation of a complex between the antibody and the polypeptide, according to which the complex is measured and is brought into relationship with the quantity of the antibody in the sample, wherein an elevated level is evaluated as indication of an allergy to the arthropod protein which the polypeptide contains.

16. An in-vetro method for measuring a cellular immune reaction, wherein a recombinant or synthetic protein or polypeptide is used, encoded by a nucleic acid
which comprises
(a) the sequence represented in SEQ ID NO: 1,
(b) a sequence deviating from a sequence according to (a) on the basis of degeneration of the genetic code,
(c) a sequence with an identity > 80 % to one of the sequences of (a) and/or (b), or
(d) a sequence which hybridises with one of the sequences according to (a),(b) and/or (c) under stringent conditions, to stimulate the cellular immune reaction.

17. A method for determining arthropod allergies in samples from the human environment, **characterised in that** the arginine kinase activity (EC 2.7.3.3) in the samples is determined.

18. A method for determining arthropod allergies in samples from the human environment, **characterised in that** the presence is determined
(a) of a nucleic acid encoding an allergenic polypeptide, comprising
(i) the sequence represented in SEQ ID NO: 1,
(ii) a sequence deviating from a sequence according to (i) on the basis of degeneration of the genetic code,
(iii) a sequence with an identity > 80 % to one of the sequences of (i) and/or (ii), or
(iv) a sequence which hybridises with one of the sequences according to (i), (ii) and/or (iii) under stringent conditions, or
(b) of an allergenic polypeptide encoded by a nucleic acid according to (a).

19. A method for determining arthropod allergies in samples from the human environment, **characterised in that** the presence of arginine kinase from Plodia interpunctella according to SEQ ID N0:2 or an arginine kinase with a sequence identity > 70 % thereto is determined.

20. A method according to Claim 19, **characterised in that** the presence of an arginine kinase from mites or moths is determined.

21. A method for preparing a medicament which comprises a synthetic or recombinant polypeptide, encoded by a nucleic acid which contains
(a) the sequence represented in SEQ ID NO: 1,
(b) a sequence deviating from a sequence according to (a) on the basis of degeneration of the genetic code,
(c) a sequence with an identity > 80 % to one of the sequences of (a) and/or (b), or
(d) a sequence which hybridises with one of the sequences according to (a),(b) and/or (c) under stringent conditions,
and can be used for the hyposensibilisation (immunotherapy) of patients with an allergy to arginine kinase from Plodia interpunctella according to SEQ ID NO:2 or an arginine kinase with a sequence identity > 70 % thereto

22. Use of arginine kinase from Plodia interpunctella according to SEQ ID NO:2 or an arginine kinase with a sequence identity > 70 % thereto for the preparation of a medicament and/or a diagnostic agent for the treatment of allergic diseases and/or for the determination of allergens.

23. Use according to Claim 22, **characterised in that** an arginine kinase from a moth or from a mite is used or determined.

24. An in-vitro method for detecting an allergy, in which arginine kinase from Plodia interpunctella according to SEQ ID NO:2 is used to determine the allergy.

25. An allergen for use as a medicament, **characterised in that** the allergen is arginine kinase from Plodia interpunctella according to SEQ ID NO:2 or an arginine kinase with a sequence identity > 70 % thereto.

## Revendications

1. Acide nucléique codant un polypeptide allergène d'arthropodes comprenant
(a) la séquence représentée dans SEQ ID NO : 1,
(b) une séquence différant d'une séquence selon (a) du fait de la dégénérescence du code génétique et/ou
(c) une séquence ayant une identité > 80 % avec l'une des séquences de (a) et/ou (b).

2. Acide nucléique comprenant un domaine qui code un polypeptide ayant la séquence représentée dans SEQ ID NO : 2.

3. Vecteur comprenant un acide nucléique selon la revendication 1 ou 2 en liaison fonctionnelle avec une séquence de contrôle de l'expression.

4. Vecteur d'expression recombinant qui possède une séquence de contrôle de l'expression qui est liée fonctionnellement à une séquence nucléotidique selon la revendication 1.

5. Cellule transformée avec un vecteur selon la revendication 3 ou 4.

6. Polypeptide allergène codé par l'un des acides nucléiques selon la revendication 1 ou 2.

7. Polypeptide allergène selon la revendication 6 ayant la séquence d'aminoacides représentée dans SEQ ID NO : 2.

8. Polypeptide allergène selon la revendication 6 ou 7 **caractérisé en ce qu'**il est fusionné avec un peptide ou polypeptide hétérologue.

9. Polypeptide allergène selon la revendication 8 **caractérisé en ce que** le peptide ou polypeptide hétérologue est un domaine liant la cellulose, la β-galactosidase ou la glutathion S-transférase.

10. Utilisation d'un polypeptide codé par un acide nucléique qui comprend
(a) la séquence représentée dans SEQ ID NO : 1,
(b) une séquence différant d'une séquence selon (a) du fait de la dégénérescence du code génétique ou
(c) une séquence ayant une identité > 80 % avec l'une des séquences de (a) et/ou (b),
comme immunogène pour la production d'anticorps.

11. Anticorps contre un polypeptide allergène selon l'une des revendications 6-9 où l'anticorps est spécifique d'un déterminant allergène du polypeptide allergène.

12. Composition destinée à être utilisée comme médicament comprenant :
(a) un acide nucléique codant un polypeptide allergène comprenant
(i) la séquence représentée dans SEQ ID NO : 1,
(ii) une séquence différant d'une séquence selon (i) du fait de la dégénérescence du code génétique,
(iii) une séquence ayant une identité > 80 % avec l'une des séquences de (i) et/ou (ii), ou
(iv) une séquence qui s'hybride avec l'une des séquences selon (i), (ii) et/ou (iii) dans des conditions stringentes,
(b) un vecteur recombinant comprenant un acide nucléique selon (a) en liaison fonctionnelle avec une séquence de contrôle de l'expression,
(c) une cellule transformée avec un vecteur selon (b),
(d) un polypeptide codé par un acide nucléique selon (a) et/ou
(e) un anticorps contre un polypeptide selon (d).

13. Utilisation d'une composition selon la revendication 12 pour la production d'un agent diagnostique et/ou thérapeutique.

14. Utilisation selon la revendication 13 pour la thérapie et/ou le diagnostic de maladies allergiques.

15. Procédé in vitro pour le diagnostic d'une allergie contre des protéines d'arthropodes où l'on met en contact un échantillon d'un liquide biologique provenant du patient, où des anticorps contre la protéine d'arthropodes sont présumés, avec un polypeptide codé par un acide nucléique qui comprend
(a) la séquence représentée dans SEQ ID NO : 1,
(b) une séquence différant d'une séquence selon (a) du fait de la dégénérescence du code génétique,
(c) une séquence ayant une identité > 80 % avec l'une des séquences de (a) et/ou (b), ou
(d) une séquence qui s'hybride avec l'une des séquences selon (a), (b) et/ou (c) dans des conditions stringentes,
dans des conditions qui permettent la formation d'un complexe entre l'anticorps et le polypeptide, après quoi le complexe est mesuré et mis en rapport avec la quantité d'anticorps dans l'échantillon, où un niveau accru est considéré comme un signe d'une allergie contre la protéine d'arthropodes qui contient le polypeptide.

16. Procédé in vitro pour la mesure d'une réaction immunitaire cellulaire où une protéine recombinante ou synthétique ou un polypeptide recombinant ou synthétique, codé(e) par un acide nucléique qui comprend
(a) la séquence représentée dans SEQ ID NO : 1,
(b) une séquence différant d'une séquence selon (a) du fait de la dégénérescence du code génétique,
(c) une séquence ayant une identité > 80 % avec l'une des séquences de (a) et/ou (b), ou
(d) une séquence qui s'hybride avec l'une des séquences selon (a), (b) et/ou (c) dans des conditions stringentes,
est utilisé(e) pour la stimulation de la réaction immunitaire cellulaire.

17. Procédé pour la détermination d'allergènes d'arthropodes dans des échantillons provenant de l'environnement de l'homme **caractérisé en ce que** l'on détermine l'activité arginine kinase (EC 2.7.3.3) dans les échantillons.

18. Procédé pour la détermination d'allergènes d'arthropodes dans des échantillons provenant de l'environnement de l'homme **caractérisé en ce que** l'on détermine la présence
(a) d'un acide nucléique codant un polypeptide allergène comprenant
(i) la séquence représentée dans SEQ ID NO: 1,
(ii) une séquence différant d'une séquence selon (i) du fait de la dégénérescence du code génétique,
(iii) une séquence ayant une identité > 80 % avec l'une des séquences de (i) et/ou (ii), ou
(iv) une séquence qui s'hybride avec l'une des séquences selon (i), (ii) et/ou (iii) dans des conditions stringentes, ou
(b) d'un polypeptide allergène codé par un acide nucléique selon (a).

19. Procédé pour la détermination d'allergènes d'arthropode dans des échantillons provenant de l'environnement de l'homme **caractérisé en ce que** l'on détermine la présence d'arginine kinase de Plodia interpunctella selon SEQ ID NO : 2 ou d'une arginine kinase ayant une identité de séquence > 70 % avec elle.

20. Procédé selon la revendication 19 **caractérisé en ce que** l'on détermine la présence d'une arginine kinase de teigne ou de mite.

21. Procédé de production d'un médicament qui contient un polypeptide synthétique ou recombinant codé par un acide nucléique qui contient
(a) la séquence représentée dans SEQ ID NO : 1,
(b) une séquence différant d'une séquence selon (a) du fait de la dégénérescence du code génétique,
(c) une séquence ayant une identité > 80 % avec l'une des séquences de (a) et/ou (b), ou
(d) une séquence qui s'hybride avec l'une des séquences selon (a), (b) et/ou (c) dans des conditions stringentes,
et qui peut être utilisé pour l'hyposensibilisation (immunothérapie) de patients ayant une allergie contre une arginine kinase de Plodia interpunctella selon SEQ ID NO : 2 ou une arginine kinase ayant une identité de séquence > 70 % avec elle.

22. Utilisation de l'arginine kinase de Plodia interpunctella selon SEQ ID NO : 2 ou d'une arginine kinase ayant une identité de séquence > 70 % avec elle pour la production d'un médicament et/ou d'un agent diagnostique pour le traitement de maladies allergiques et/ou pour la détermination d'allergènes.

23. Utilisation selon la revendication 22 **caractérisée en ce qu'**une arginine kinase de la mite ou de la teigne est utilisée ou déterminée.

24. Procédé in vitro pour la mise en évidence d'une allergie dans lequel une arginine kinase de Plodia interpunctella selon SEQ ID NO : 2 est utilisée pour la détermination de l'allergie.

25. Allergène destiné à être utilisé comme médicament **caractérisé en ce que** l'allergène est une arginine kinase de Plodia interpunctella selon SEQ ID NO : 2 ou une arginine kinase ayant une identité de séquence > 70 % avec elle.
